# EUROPEAN PATENT APPLICATION

(11) **EP 4 725 546 A1**
(43) Date of publication of application: **15.04.2026**
(21) Application number: 25207789.6
(22) Date of filing: 09.10.2025
(51) Int. Cl.: A61N 1/39, A61B 5/053, A61B 5/00

(54) **MANAGING IMPEDANCE SIGNAL INTERFERENCE**

(30) Priority: 09.10.2024 US 202463705477 P; 05.02.2025 US 202563754522 P; 07.10.2025 US 202519352337
(71) Applicant: Stryker Corporation, Portage, MI 49002 (US)
(72) Inventor: LINVILLE, David J., Portage, 49002 (US); APPERSON, Ryan William, Portage, 49002 (US); CHAPMAN, Fred W., Portage, 49002 (US); NORDESS, Rocky, Portage, 49002 (US); SHARIF, Reza, Portage, 49002 (US); TAYLOR, Tyson G., Portage, 49002 (US); WALKER, Robert G., Portage, 49002 (US); YIN, Rose Tingwei, Portage, 49002 (US); MARX, Robert P., Portage, 49002 (US); CHAIMANONART, Nattapon, Portage, 49002 (US)
(74) Representative: V.O.

(57) **Abstract**

An example method includes detecting, at a first set of electrodes disposed on a subject, a first interrogation signal having a first carrier frequency. The example method further includes detecting, in data representing the first interrogation signal, an artifact associated with a second interrogation signal having a second carrier frequency; removing, from the data representing first interrogation signal, the artifact; and determining, by analyzing the data representing the first interrogation signal, a transthoracic impedance of the subject.

## Description

### CROSS-REFERENCE TO RELATED APPLICATIONS

This application claims the priority of U.S. Patent App. No. 19/352,337, which was filed on October 7, 2025, which claims priority to U.S. Provisional App. No. 63/705,477, which was filed on October 9, 2024; and U.S. Provisional App. No. 63/754,522, which was filed on February 5, 2025, each of which is incorporated by reference herein in its entirety.

### BACKGROUND

Particular heart arrhythmias, such as ventricular fibrillation (VF) and pulseless ventricular tachycardia (VT), are deadly if untreated. An individual with one of these arrhythmias can be treated by administering an electrical shock to the individual's heart. This treatment is known as defibrillation. The arrhythmias that are treatable by defibrillation, or other types of electrotherapies, are known as shockable arrhythmias.

Unfortunately, many instances of shockable arrhythmias are resistant to conventional defibrillation therapies. For instance, an individual is determined to have refractory VF if they have VF that continues through the administration of an electrical shock. Recently, double sequential defibrillation (DSD) (also referred to as "double sequential external defibrillation" or "DSED") has been proposed as an alternative to single-shock defibrillation. A DSD therapy, for instance, can be administered by discharging two electrical shocks to an individual's heart, rather than one. Researchers have suggested that DSD administration can increase survivability of refractory VF. Cheskes et al., 387 N. Engl. J. Med. 1947 (2022). A DSD therapy, in some cases, is administered by two defibrillators that are simultaneously connected to the individual.

### SUMMARY

According to an aspect is provided a method, comprising: receiving data representing a first interrogation signal transmitted through a subject by a first impedance-measuring device, the first interrogation signal having a first carrier frequency; determining that the data representing the first interrogation signal comprises an artifact associated with a second interrogation signal having a second carrier frequency; and in response to determining that the data representing the first interrogation signal comprises the artifact, determining that the subject is connected to a second impedance-measuring device.

Optionally, the artifact is a first artifact, wherein the data representing the first interrogation signal is representative of a first electrical signal detected from electrodes disposed on the subject, wherein the method further comprises: in response to determining that the data representing the first interrogation signal comprises the artifact, causing suppression of the first interrogation signal; in response to causing suppression of the first interrogation signal, receiving data representing a second electrical signal detected from the electrodes disposed on the subject; and determining that the data representing the second electrical signal comprises a second artifact associated with the second interrogation signal having the second carrier frequency, and wherein determining that the subject is connected to the second impedance-measuring device is further in response to determining that the data representing the second electrical signal comprises the second artifact.

Optionally, the method further comprises: in response to determining that the subject is connected to the second impedance-measuring device, outputting a notification to a user.

Optionally, the method further comprises: detecting an input signal from the user confirming that the subject is connected to the second impedance-measuring device; and in response to detecting the input signal, suppressing an impedance-dependent function of the first impedance-measuring device.

Optionally, the notification indicates a presence of the second impedance-measuring device or a warning indicating a potential malfunction of the second impedance-measuring device.

Optionally, the method further comprises: in response to determining that the subject is connected to the second impedance-measuring device: identifying a communication window associated with the second impedance-measuring device; and causing the first impedance-measuring device to suppress the first interrogation signal during the communication window.

Optionally, the method further comprises: in response to determining that the subject is connected to the second impedance-measuring device, causing the first impedance-measuring device to suppress a function utilizing the first interrogation signal, the function comprising: analyzing an ECG of the subject; removing an artifact from the ECG of the subject; detecting chest compressions performed on the subject; detecting ventilation performed on the subject, detecting spontaneous respiration of the subject, or customizing a parameter of an electrical shock to be administered to the subject.

Optionally, the method further comprises: removing, from the data indicative of the first interrogation signal, the artifact; and determining, by analyzing the data indicative of the first interrogation signal, a transthoracic impedance of the subject.

Optionally, the method further comprises: determining a type of the second impedance-measuring device by analyzing the artifact; in response to determining the type of the second impedance-measuring device, selecting a subsequent action; and performing the subsequent action.

Optionally, selecting the subsequent action comprises selecting among: suppressing the first interrogation signal; outputting a notification indicating the type of the second impedance-measuring device; pairing with the second impedance-measuring device; removing the artifact from the first interrogation signal; and deactivating a function utilizing the first interrogation signal.

Optionally, determining the type of the second impedance-measuring device comprises: determining a carrier frequency of the second interrogation signal by analyzing the first interrogation signal; and determining the type of the second impedance-measuring device by analyzing the carrier frequency, and wherein the method further comprises: determining, by analyzing the type of the second impedance-measuring device, that there is a risk of damage to the first impedance-measuring device, of damage to the second-impedance measuring device, or of damage to the subject.

Optionally, the method further comprises: in response to determining that the subject is connected to the second impedance-measuring device, causing the first impedance-measuring device to initiate a self-test.

Optionally, causing the first impedance-measuring device to initiate the self-test comprises causing the first impedance-measuring device to initiate the self-test in response to receiving a request to power down the first impedance-measuring device.

Optionally, the method further comprises: storing, in memory, an indication that the subject is connected to the second impedance-measuring device.

According to an aspect is provided a computing device, comprising: a processor configured to: receive data representing a first interrogation signal transmitted through a subject by a first impedance-measuring device, the first interrogation signal having a first carrier frequency; detect, in the data representing the first interrogation signal, an artifact associated with a second interrogation signal having a second carrier frequency; in response to detecting the artifact, determine that the subject is connected to a second impedance-measuring device.

Optionally, the computing device further comprises: an output device; wherein the processor is further configured to, in response to determining that the subject is connected to the second impedance-measuring device, cause the output device to output a notification.

According to an aspect is provided a monitor-defibrillator, comprising: a first impedance-measuring device configured to transmit a first interrogation signal through a subject, the first interrogation signal having a first carrier frequency; and the computing device as described herein.

Optionally, the artifact is a first artifact, wherein the data representing the first interrogation signal is representative of a first electrical signal detected from electrodes disposed on the subject, wherein the processor is further configured to: in response to determining that the data representing the first interrogation signal comprises the artifact, causing suppression of the first interrogation signal; in response to causing suppression of the first interrogation signal, receive data representing a second electrical signal detected from the electrodes disposed on the subject; and determine that the data representing the second electrical signal comprises a second artifact associated with the second interrogation signal having the second carrier frequency, and wherein determining that the subject is connected to the second impedance-measuring device is further in response to determining that the data representing the second electrical signal comprises the second artifact.

Optionally, the processor is further configured to: detect an input signal from the user confirming that the subject is connected to the second impedance-measuring device; and in response to detecting the input signal, suppress an impedance-dependent function of the first impedance-measuring device.

Optionally, the notification indicates a presence of the second impedance-measuring device or a warning indicating a potential malfunction of the second impedance-measuring device.

Optionally, the processor is further configured to: in response to determining that the subject is connected to the second impedance-measuring device: a) identify a communication window associated with the second impedance-measuring device, and cause the first impedance-measuring device to suppress the first interrogation signal during the communication window; and/or b) cause the first impedance-measuring device to suppress a function utilizing the first interrogation signal, the function comprising: analyzing an ECG of the subject; removing an artifact from the ECG of the subject; detecting chest compressions performed on the subject; detecting ventilation performed on the subject, detecting spontaneous respiration of the subject, or customizing a parameter of an electrical shock to be administered to the subject; and/or c) output, to the subject, a first electrical shock having a predetermined voltage; determining that the second impedance-measuring device is separated from the subject; in response to determining that the second impedance-measuring device is separated from the subject: determining a transthoracic impedance of the subject by analyzing the first interrogation signal; determining a voltage of a second electrical shock by analyzing the transthoracic impedance; and outputting the second electrical shock.

Optionally, the processor is further configured to: remove, from the data indicative of the first interrogation signal, the artifact; and determine, by analyzing the data indicative of the first interrogation signal, a transthoracic impedance of the subject.

Optionally, the processor is further configured to: determine a type of the second impedance-measuring device by analyzing the artifact; in response to determining the type of the second impedance-measuring device, select a subsequent action; and cause performance of the subsequent action; wherein the subsequent action can e.g. be selected from: suppressing the first interrogation signal; outputting a notification indicating the type of the second impedance-measuring device; pairing with the second impedance-measuring device; removing the artifact from the first interrogation signal; and deactivating a function utilizing the first interrogation signal.

Optionally, determining the type of the second impedance-measuring device comprises: determining a carrier frequency of the second interrogation signal by analyzing the first interrogation signal; and determining the type of the second impedance-measuring device by analyzing the carrier frequency; and optionally, determining, by analyzing the type of the second impedance-measuring device, that there is a risk of damage to the first impedance-measuring device, of damage to the second-impedance measuring device, or of damage to the subject.

Optionally, the processor is further configured to: in response to determining that the subject is connected to the second impedance-measuring device, causing the first impedance-measuring device to initiate a self-test; wherein optionally causing the first impedance-measuring device to initiate the self-test comprises causing the first impedance-measuring device to initiate the self-test in response to receiving a request to power down the first impedance-measuring device.

Optionally, the processor is further configured to: store, in memory, an indication that the subject is connected to the second impedance-measuring device.

Optionally, the first impedance-measuring device comprises a detection circuit configured to: output, to electrodes configured to be disposed on a subject, a first interrogation signal having a first carrier frequency and a first current amplitude; detect a voltage of the first interrogation signal; and detect a first electrical shock output to the subject; the monitor-defibrillator further comprising: a treatment circuit configured to output a second electrical shock to the electrodes; and a display; wherein the processor is configured to: detect, in data representing the voltage of the first interrogation signal, the artifact associated with the second interrogation signal having the second carrier frequency; determine, by analyzing the second carrier frequency, that the subject is connected to an automated external defibrillator (AED); and in response to determining that the subject is connected to the AED: cause the detection circuit to suppress the first interrogation signal; cause the display to output a notification indicating the AED; and cause the treatment circuit to output the second electrical shock in response to the detection circuit detecting the first electrical shock.

Optionally, the processor is configured to detect the artifact associated with the second interrogation signal by: converting the data representing the voltage of the first interrogation signal from a time domain to a frequency domain; and in response to converting the data representing the voltage of the first interrogation signal from the time domain to the frequency domain, determining that an amplitude of the data corresponding to the second carrier frequency is above a threshold.

Optionally, the monitor-defibrillator further comprises: an input device configured to receive, from a user, an input signal selecting a multi-shock mode, wherein the processor is configured to cause the treatment circuit to output the second electrical shock further in response to the input device receiving the input signal.

It will be appreciated that all options and features can be combined.

### BRIEF DESCRIPTION OF THE DRAWINGS

FIG. 1 illustrates an example environment for accommodating and managing multiple impedance-measuring devices monitoring the same subject.
FIG. 2 illustrates example signaling between a medical device and an impedance-measuring device using impedance interrogation signals.
FIG. 3 illustrates an example of an impedance interrogation signal.
FIG. 4 illustrates an example process for adapting a frequency of an impedance interrogation signal based on the presence of an impedance-measuring device.
FIG. 5 illustrates an example process for disabling functions of a medical device based on the presence of an impedance-measuring device.
FIG. 6 illustrates an example process for removing, from data representing a first interrogation signal, an artifact caused by a second interrogation signal.
FIGS. 7A and 7B illustrate examples of simulated impedance signals.
FIG. 8 illustrates an example of an external defibrillator configured to perform various functions described herein.
FIGS. 9A and 9B illustrate examples of environments and timing related to administering a DSD therapy. FIG. 9A shows an environment configured to administer the DSD therapy. FIG. 9B shows a timing relationship of multiple shocks administered in the DSD therapy.

### DETAILED DESCRIPTION

Various implementations described herein relate to techniques for utilizing and managing impedance interrogation signals output by multiple medical devices to a subject. For example, some implementations described herein are suitable for avoiding inaccurate impedance measurements by a defibrillator when another impedance-measuring device is connected to the same subject. In some cases, implementations of the present disclosure are utilized when multiple defibrillators are connected to the same subject to coordinate administration of a multi-shock therapy, such as a DSD therapy, to the subject.

In previous techniques, a defibrillator is configured to detect an impedance (e.g., a transthoracic impedance) of a subject by outputting an impedance interrogation signal to electrodes disposed on the skin of the subject. The impedance interrogation signal, for example, is a periodic signal having a predetermined frequency. In some cases, the predetermined frequency is a carrier frequency. However, if another impedance-measuring device outputs another impedance interrogation signal to electrodes disposed on the skin of the subject with the same or similar frequency, the other impedance interrogation signal may cause the defibrillator to inaccurately identify the impedance of the subject. Inaccurate impedance measurements can interfere with various functions of the defibrillator, such as chest compression detection, ventilation detection, voltage compensation for defibrillation waveforms, and the like.

Various implementations of the present disclosure address these and other problems. For example, a defibrillator detects when another impedance-measuring device is being used, or about to be used, on the same subject as the defibrillator. In some cases, the defibrillator detects when the impedance-measuring device is within the vicinity of the defibrillator. In some cases, the defibrillator detects when the impedance-measuring device is connected to the same subject as the defibrillator. In response, the defibrillator, in some cases, is configured to specifically select a frequency of an impedance interrogation signal such that it is distinct from the frequency of an electrical signal output by the impedance-measuring device. In some examples, the defibrillator is configured to deactivate one or more impedance-dependent functions of the defibrillator. According to some implementations, the defibrillator is configured to remove, from the impedance interrogation signal, interference caused by the impedance-measuring device. In some cases, the defibrillator and the impedance-measuring device are configured to communicate via impedance interrogation signals transmitted through the body of the subject. Accordingly, implementations of the present disclosure prevent inaccurate measurements and malfunctions by the defibrillator when another impedance-measuring medical device is connected to the same subject. In some cases, these techniques can enhance the application of a DSD therapy administered by the defibrillator and the impedance-measuring device, which may be another defibrillator connected to the subject.

Implementations of the present disclosure will now be described with reference to the accompanying figures.

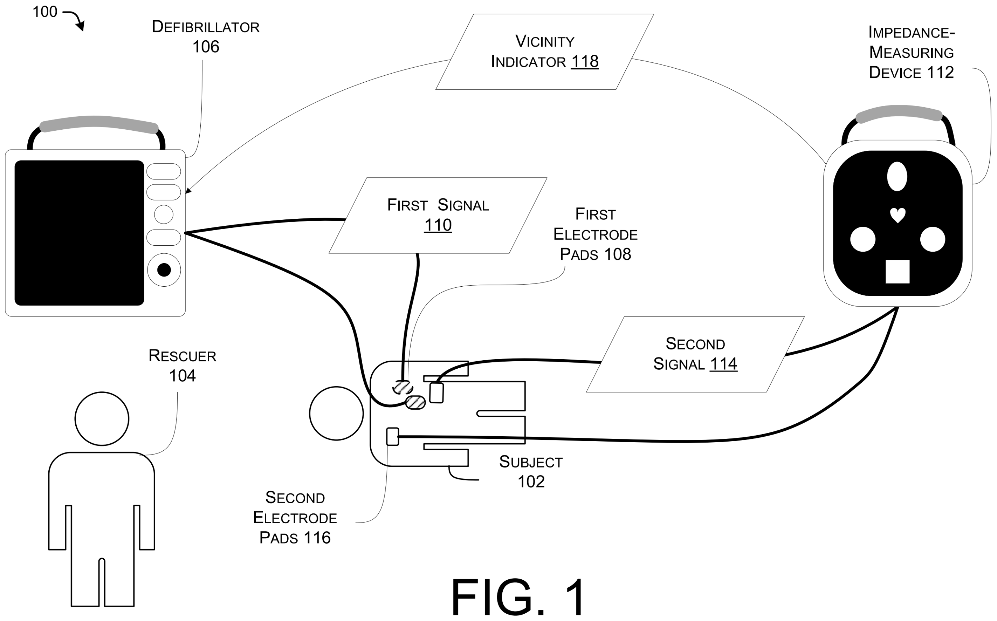

FIG. 1 illustrates an example environment 100 for accommodating and managing multiple impedance-measuring devices monitoring the same subject 102. In various cases, the environment 100 is in an out-of-hospital environment. For example, the subject 102 may have experienced a medical emergency in a non-hospital, public space, such as a library, airport terminal, school, or office building. In some cases, the subject 102 has collapsed or otherwise lost consciousness within the environment 100. In some examples, the subject 102 has experienced one or more other types of symptoms associated with a serious health condition.

According to some examples, a rescuer 104 is present in the environment 100 to provide medical assistance to the subject 102. In some cases, the rescuer 104 has specialized medical knowledge. For instance, the rescuer 104 may be an emergency medical services (EMS) provider, a physician, a nurse, or some other type of clinical care provider.

In various implementations, the rescuer 104 operates a defibrillator 106 configured to monitor and treat the subject 102. The defibrillator 106 is a type of medical device. In some cases, the defibrillator 106 is a monitor-defibrillator. In some examples, the defibrillator 106 is an automated external defibrillator (AED). In various cases, the defibrillator 106 is a portable device. For example, the defibrillator 106 includes an on-board power source (e.g., one or more batteries) configured to supply power to circuitry within the defibrillator 106. In some examples, the defibrillator 106 is carried into the environment 100 by the rescuer 104.

In particular cases, the defibrillator 106 is electrically connected to first electrode pads 108 disposed on the skin of the subject 102. The electrode pads 108 include electrodes configured to receive electrical signals from the subject 102 and/or to output electrical signals to the subject 102. In some cases, the electrode pads 108 include electrocardiogram (ECG) electrodes configured to detect an electrical signal output by the heart of the subject 102. Based on this electrical signal, the defibrillator 106 detects the ECG of the subject 102. In some cases, the electrode pads 108 include electrodes configured to output one or more types of therapy signals to the subject 102. Optionally, the defibrillator 106 outputs pacing pulses, electrical shocks, or any other type of electrotherapy signal that can be used to treat the cardiac condition of the subject 102.

According to some examples, the defibrillator 106 is configured to detect an electrical impedance of at least a portion of the subject 102 using the first electrode pads 108. For example, the defibrillator 106 outputs, to electrodes in the first electrode pads 108, a first signal 110 that induces an electrical current between the first electrode pads 108. According to various implementations, the first signal 110 has a first frequency. For example, the first signal 110 includes a carrier signal and/or a message signal, wherein the first frequency represents a carrier frequency of the carrier signal. Optionally, the message signal includes information encoded by modulating the carrier signal. For example, the first signal 110 may have an additional frequency band that is modulated on the carrier at a frequency different than an impedance frequency, and could contain data to be communicated between the defibrillator 106 and a separate device electrically coupled with the subject 102. In some cases, the data canbe communicated back and forth between devices, and used to transmit patient data, device identification, or may be used to negotiate carrier frequencies between the devices.

The electrical current induced between the first electrode pads 108 by the first signal 110, for example, may be lower than a predetermined level to avoid harm to the subject 102. According to various cases, the predetermined level of current imposed by the first signal 110 is dependent on its frequency. For example, if the first signal 110 is a direct current (DC) signal, then the first signal 110 is output to induce a current of 50 microamps (µA) or less. If the first signal 110 has a frequency of 100 kilohertz (kHz), then the first signal 110 is output to induce a current of 1 mA root mean square (RMS) or less. The current limit through the subject 102 may be specified by an applicable regulatory agency, such as the US Food and Drug Administration (FDA) if the defibrillator 106 is operated in the US. In various cases, the first frequency of the first signal 110 is in a range of 5 Hz to 1 MHz, a range of 500 Hz to 1 MHz, or a range of 10 kHz to 100 kHz.

The defibrillator 106 is configured to detect the electrical impedance of the portion of the subject 102 between the first electrode pads 108 by determining the voltage necessary to induce the first signal 110 at a predetermined current. In various cases, an electrical path between the first electrode pads 108 extends through a portion of the chest of the subject 102. Accordingly, the electrical impedance detected by the defibrillator 106 is a transthoracic impedance of the subject 102.

In various implementations, the defibrillator 106 is configured to output the first signal 110 at a current that varies over time in accordance with the carrier frequency. For instance, the amplitude of the current of the first signal 110 output by the defibrillator 106 over time may vary as a periodic waveform (e.g., a sine wave, a square wave filtered with various analog filter circuits within the defibrillator 106, etc.) that has the carrier frequency. The defibrillator 106, in various cases, may be configured to detect the voltage of the first signal 110 that is utilized to achieve the waveform of the current of the first signal 110 over time. The impedance along the path of the first signal 110 through the subject 102 is correlated to the amplitude of the voltage of the first signal 110, for example.

Notably, in some cases, the defibrillator 106 may simultaneously detect a voltage indicative of an ECG of the subject 102 while detecting the voltage of the first signal 110. For example, the defibrillator 106 is configured to detect the voltage between the first electrode pads 108 over time. The defibrillator 106, for instance, is configured to split the detected voltage into two paths within a detection circuit of the defibrillator 106. The first path is an ECG detection path, and includes at least one filter configured to remove a high-frequency portion (e.g., 500 Hz to 1 MHz) of the detected signal corresponding to the carrier frequency of the first signal 110. In some cases, the ECG detection path obtains a portion of the detected voltage defined in a range of 0.05 Hz to 500 Hz, while excluding other frequencies of the detected voltage. The second path, for instance, is an impedance detection paths, and includes at least one filter configured to remove a low-frequency band (e.g., a band in a range of 70 Hz to 500 Hz) that corresponds to the ECG. According to various implementations, the second path includes a quadrature filter that extracts the phase and amplitude of the carrier signal, which are the result of the real and reactive impedances of the subject 102. In some cases, an impedance of the subject 102 is calculated based the real and reactive impedances of the subject 102. In various implementations, a motion artifact may be in a range of 0.05 Hz to 70 Hz. Optionally, the defibrillator 106 includes a third path corresponding to the motion artifact frequency range. Accordingly, in some examples, the defibrillator 106 can detect a motion artifact by detecting an artifact in the 0.05 to 70 Hz range of the signal on the impedance path and/or the motion artifact path. That motion artifact, in various cases, may also be present in the signal on the ECG path. Accordingly, in some implementations, the motion artifact on the ECG path can be removed and/or accounted for based on detection performed on the impedance path.

The electrical impedance, for example, changes over time. According to various cases, the defibrillator 106 is configured to sample the electrical impedance at a sampling frequency. In some examples, the sampling frequency is in a range of 5 Hz to 1 MHz, such as a range of 10 kHz to 125 kHz. In various implementations, the sampling frequency is at least double the frequency of the first frequency and/or the frequency of a signal detected from the first signal 110. However, a higher sampling rate (e.g., ten times that of the first frequency) can facilitate filtering and artifact removal.

The electrical impedance is an example of a physiological parameter detected by the defibrillator 106. In various implementations, the defibrillator 106 is configured to infer one or more additional parameters based on the electrical impedance. According to some cases, the electrical impedance increases as lungs of the subject 102 fill with air (e.g., inhalation), and decreases as the air is expelled from the lungs of the subject 102. If the subject 102 is spontaneously breathing, the defibrillator 106 is, in some cases, configured to detect a respiration rate of the subject 102 by detecting a frequency of peaks and/or troughs in the electrical impedance. If the subject 102 is receiving positive pressure ventilation, such as by a mechanical ventilator or a bag-valve mask (BVM) operated by the rescuer 104, the defibrillator 106 is configured to detect a ventilation rate applied to the subject 102 by detecting the frequency of peaks and/or troughs in the electrical impedance.

The electrical impedance additionally varies based on the application of compressions to the chest of the subject 102, such as during the administration of cardiopulmonary resuscitation (CPR). For example, the electrical impedance decreases as an individual compression is applied to the chest of the subject 102 and increases when the compression is released from (or if active decompression is applied to) the chest of the subject 102. Accordingly, in some cases, the defibrillator 106 is configured to detect a rate of chest compressions applied to the subject 102 by determining a frequency of peaks and/or troughs in the electrical impedance.

The defibrillator 106 is configured to perform one or more functions based, at least in part, on the electrical impedance of the subject 102. These functions, for instance, can be referred to as "impedance-dependent functions."

In some implementations, the defibrillator 106 has a ventilation coaching function, wherein the defibrillator 106 determines a ventilation rate of the subject 102 based on the impedance, compares the ventilation rate to a target range, and outputs an instruction to maintain or adjust the ventilation rate based on whether the ventilation rate is in the target range. For instance, if the ventilation rate is lower than a threshold, the defibrillator 106 instructs the rescuer 104 to increase the ventilation rate.

In some cases, the defibrillator 106 has a chest compression coaching function, wherein the defibrillator 106 determines a rate of chest compressions applied to the subject 102 based on the impedance, compares the chest compression rate to a target range, and outputs an instruction to maintain or adjust the rate of the chest compressions based on whether the rate is in the target range. For example, if the rate of the chest compressions is higher than a threshold, the defibrillator 106 instructs the rescuer 104 to lower the rate of the chest compressions. The defibrillator 106, in some instances, is configured to provide other types of chest compression feedback. For example, if a difference between a minimum and a maximum of the transthoracic impedance during a time interval is less than a threshold, the defibrillator 106 instructs the rescuer 104 to increase a chest compression depth and/or change a position at which the chest compressions are applied to the subject 102.

According to some cases, the defibrillator 106 has a shock advisory function that is dependent on the electrical impedance. For example, the defibrillator 106 is configured to analyze an ECG of the subject 102 in order to determine whether the ECG is indicative of a shockable arrhythmia that is treatable by administration of an electrotherapy. Examples of shockable arrhythmias, for instance, include VF and pulseless VT, which are treatable by the administration of at least one electrical shock. Other examples of shockable arrhythmias include atrial fibrillation (AF), which is treatable by the administration of synchronized cardioversion (e.g., the administration of one or more electrical shocks that are temporally overlapping with at least one QRS complex and/or R-wave, or are administered during intervals defined between at least one QRS complex and at least one T-wave), as well as bradycardia, which is treatable by the administration of pacing pulses.

If the subject 102 is receiving a treatment when the ECG is detected, the ECG includes an artifact associated with the treatment. In various cases, the defibrillator 106 is configured to remove the artifact from the ECG, or otherwise analyze the ECG in view of known characteristics of the artifact, to accurately assess whether the shockable rhythm is present. According to some examples, the defibrillator 106 removes or identifies the artifact based on the detected impedance. For example, the defibrillator 106 is configured to determine a rate of chest compressions applied to the subject 102 based on the transthoracic impedance, and then identify or remove a chest compression artifact in the ECG based on the determined rate. In various cases, the defibrillator 106 is configured to determine a ventilation rate applied to the subject 102 based on the transthoracic impedance, and then identify or remove a ventilation artifact in the ECG based on the determined rate. In some cases, the defibrillator 106 selects a digital filter with a reject band that includes the determined rate of the ventilation and/or chest compressions. Once the treatment artifact has been removed (or reduced) from the ECG, the defibrillator 106 analyzes the ECG for the presence of the shockable arrhythmia. In some cases, the defibrillator 106 outputs a recommendation to administer an electrical shock to the subject 102 if the defibrillator 106 determines that the shockable arrhythmia is present in the ECG and/or there is greater than a threshold likelihood that the shockable arrhythmia is present in the ECG.

In various cases, the defibrillator 106 executes a motion detection function based on the electrical impedance. For example, if the defibrillator 106 detects artifact(s) in the electrical impedance over time that are irregularly timed, such that they are not consistent with respiration, ventilation, or chest compressions, the defibrillator 106 may determine that the subject 102 is moving. In some cases, the defibrillator 106 additionally includes an accelerometer. If the defibrillator 106 determines that an acceleration detected by the accelerometer is temporally consistent with (e.g., temporally overlapping with) the artifact in the impedance, then the defibrillator 106 infers that both the subject 102 and the defibrillator 106 are in motion. For instance, the subject 102 and the defibrillator 106 are moving within a patient transport vehicle (e.g., ambulance). If the defibrillator 106 determines that the acceleration detected by the accelerometer is minimal, or is inconsistent with the artifact in the impedance, then the defibrillator 106 infers that the subject 102 is moving independently of the defibrillator 106. For example, the subject 102 may be spontaneously moving, experiencing a seizure, or receiving a treatment (e.g., from the rescuer 104). The defibrillator 106, in some cases, reports the detected motion to the rescuer 104. In some implementations, the defibrillator 106 selectively deactivates one or more additional functions (e.g., the shock advisory function) in response to detecting the motion.

According to various cases, the defibrillator 106 is configured to determine a parameter of a treatment to be administered to the subject 102 based, at least in part, on the electrical impedance. For example, the defibrillator 106 is configured to determine an energy level, a voltage level, a current, a duration, a shape, a vector, or some other characteristic of an electrical shock to be administered to the subject 102, based on the transthoracic impedance. The defibrillator 106, in various cases, is configured to perform voltage compensation of the treatment based, at least in part, on the electrical impedance. If the transthoracic impedance is relatively high, for instance, the defibrillator 106 may output the electrical shock at a relatively high voltage and/or a relatively high duration in order to achieve a desired energy level (e.g., 200 J, 360 J, or the like) to be administered to the heart of the subject 102. That is, the defibrillator 106 may be configured to adjust a parameter of the electrical shock to compensate for the detected transthoracic impedance.

The functionality of the defibrillator 106 is enhanced by detecting the impedance of the subject 102 using the first signal 110, in various cases. However, in some cases, the detected impedance of the subject 102 is incorrect due to the presence of a separate impedance-measuring device 112. For instance, the impedance-measuring device is within the vicinity of the subject 102 and/or defibrillator 106. In some cases, the impedance-measuring device is electrically connected to the subject 102 and/or the defibrillator 106. In some examples, the impedance-measuring device 112 is a medical device, such as an additional defibrillator configured to administer a treatment to the subject 102. For example, the impedance-measuring device 112 is an AED that is configured to monitor and/or treat the subject 102. In some cases, the impedance-measuring device 112 is a wearable defibrillator, a wearable patient monitor, an implantable device, or any combination thereof.

In particular cases, the impedance-measuring device 112 is utilized to provide a coordinated therapy to the subject 102 with the defibrillator 106. In some cases, the subject 102 is exhibiting a condition that is treatable by a sequential-shock therapy. Collectively, the defibrillator 106 and the impedance-measuring device 112 are configured to administer the sequential shock therapy to the subject 102. For example, the subject 102 is exhibiting a condition (e.g., refractory VF) that is treatable by a multi-shock therapy (e.g., a DSD therapy). The defibrillator 106, for example, is configured to output a primary shock in the DSD therapy, and the impedance-measuring device 112 is configured to output a secondary shock in the DSD therapy. In various cases, the rescuer 104 intentionally activates both the defibrillator 106 and the impedance-measuring device 112, in order to enable the administration of the coordinated therapy.

In some examples, the impedance-measuring device 112 is incidentally present in the environment 100. For instance, the impedance-measuring device 112 may have performed initial monitoring and/or treatment of the subject 102 prior to the arrival of the rescuer 104 in the environment 100. Upon reaching the subject 102, the rescuer 104 may have applied the defibrillator 106 to the subject 102 without deactivating the existing impedance-measuring device 112. In some examples, the impedance-measuring device 112 is an implantable device (e.g., an implantable pacemaker or defibrillator) that is disposed underneath the skin of the subject 102.

The impedance-measuring device 112 is configured to transmit a second signal 114 to the subject 102 via second electrode pads 116 disposed on the skin of the subject 102. In various cases, the impedance-measuring device 112 detects an impedance of the subject 102 along an electrical path between the second electrode pads 116 based, at least in part, on the second signal 114. For example, the second signal 114 induces an electrical current between the second electrode pads 116. According to various implementations, the second signal 114 has a second frequency. For instance, the second signal 114 includes a carrier signal and/or a message signal, wherein the second frequency is a carrier frequency of the carrier signal. In some cases, the message signal encodes data via modulation of the carrier signal. The electrical current induced between the second electrode pads 116 by the second signal 114, for example, may be lower than a predetermined level to avoid harm to the subject 102. According to various cases, the predetermined level of current imposed by the second signal 114 is dependent on its frequency. For example, if the second signal 114 is a DC signal, then the second signal 114 is output to induce a current of 50 µA (or 10 µA) or less. If the second signal 114 has a frequency of 100 kHz, then the second signal 114 is output to induce a current of 1 mA RMS or less. In various cases, the first frequency of the first signal 110 is in a range of 5 Hz to 100 kHz. The impedance-measuring device 112 is configured to detect the electrical impedance of the portion of the subject 102 between the second electrode pads 116 by determining the voltage necessary to induce the second signal 114 at a predetermined current. In various cases, an electrical path between the second electrode pads 116 extends through a portion of the chest of the subject 102. Accordingly, the electrical impedance detected by the impedance-measuring device 112 is a transthoracic impedance of the subject 102. The impedance-measuring device 112, for instance, is configured to perform one or more of the impedance-dependent functions described above based on the electrical impedance detected by the impedance-measuring device 112.

In some cases, however, the second signal 114 output by the impedance-measuring device 112 decreases the accuracy of the impedance detected by the defibrillator 106. For example, if the first signal 110 and the second signal 114 are output at similar frequencies, then the defibrillator 106 may detect an inaccurate electrical impedance across the first electrode pads 108. The inaccurate electrical impedance, for instance, can interfere with the safe and accurate execution of the impedance-dependent functions of the defibrillator 106.

Similarly, the first signal 110 can interfere with the second signal 114, such that the first signal 110 may prevent the impedance-measuring device 112 from accurately detecting the impedance of the subject 102 across the second electrode pads 116. This interference can prevent the impedance-measuring device 112 from safely or accurately executing one or more impedance-dependent functions of the impedance-measuring device 112. For instance, the impedance-measuring device 112 may not be designed to operate safely when the first signal 110 is transmitted and/or the defibrillator 106 is electrically connected with the subject 102.

Various implementations of the present disclosure relate to techniques for preventing the defibrillator 106 from malfunctioning or becoming damaged due to the second signal 114 output by the impedance-measuring device 112. Moreover, various implementations of the present disclosure relate to techniques for preventing the impedance-measuring device 112 from malfunctioning or becoming damaged due to the first signal 110 output by the defibrillator 106. In various cases, the defibrillator 106 is configured to detect that the impedance-measuring device 112 is within the vicinity of the defibrillator 106. The term "vicinity," as used herein, may refer to devices within a threshold distance of each other, communicatively coupled with each other, electrically coupled with each other, connected to the same subject, or any combination thereof.

In some cases, the defibrillator 106 is configured to detect the presence of the impedance-measuring device 112 by detecting and analyzing the second signal 114. For example, the defibrillator 106 detects the second signal 114 by detecting an electrical signal at the first electrode pads 108. According to various cases, the defibrillator 106 is configured to recognize that the impedance-measuring device 112 is within the vicinity of the defibrillator 106 by detecting, in the electrical signal, a characteristic associated with an impedance-interrogation signal. For example, if the electrical signal includes a frequency component that is above a threshold and is consistent with an impedance-interrogation signal (e.g., a frequency within a range of 5 Hz to 1 MHz), then the defibrillator 106 may infer that the impedance-measuring device 112 is connected to the subject 102. In some cases, the defibrillator 106 is configured to identify the presence of the impedance-measuring device 112 by analyzing an impedance waveform that it detects from the first electrode pads 108, and classifying the impedance waveform as a waveform associated with multiple impedance interrogation signals at respective frequencies that differ by no more than the threshold range.

In some examples, the defibrillator 106 detects the presence of the impedance-measuring device 112 by being communicatively coupled with the impedance-measuring device 112. For example, the defibrillator 106 is configured to identify that the impedance-measuring device 112 is within the vicinity of the defibrillator 106 by receiving a vicinity indicator 118 from the impedance-measuring device 112. The vicinity indicator 118 is transmitted over one or more communication interfaces. The communication interfaces include one or more wired interfaces (e.g., Ethernet, optical, electrical, etc.), one or more wireless interfaces (e.g., BLUETOOTH^{™}, Near-Field Communication (NFC), WI-FI^{™}, cellular, etc.), or a combination thereof. In some cases, the vicinity indicator 118 indicates that the impedance-measuring device 112 is transmitting, or is going to transmit, the second signal 114. In some examples, the vicinity indicator 118 indicates one or more characteristics of the second signal 114, such as a frequency, phase, or amplitude of the second signal 114.

The defibrillator 106 is configured to perform one or more actions in response to detecting the impedance-measuring device 112. In some examples, the defibrillator 106 refrains from transmitting the first signal 110, or detecting the electrical impedance of the subject 102, in response to detecting that the impedance-measuring device 112 is within the vicinity of the defibrillator 106. In various implementations, the defibrillator 106 is configured to disable one or more of the impedance-dependent functions in response to detecting the presence of the impedance-measuring device 112 in the vicinity of the defibrillator 106. In some implementations, the defibrillator 106 outputs an alert and/or prompt indicating the presence of the impedance-measuring device 112 and/or a risk that the impedance-dependent functions will operate incorrectly due to the presence of the impedance-measuring device 112. The defibrillator 106, for instance, may output one or more notifications based on detecting that the impedance measuring device 112 is coupled with the subject 102. In various cases, the defibrillator 106 disables the impedance-dependent function(s) in response to receiving an input signal from the rescuer 104. Accordingly, the defibrillator 106 is prevented from relying on an incorrect impedance of the subject 102 when monitoring and/or treating the subject 102.

In some examples, the defibrillator 106 is configured to modify the first signal 110 in response to detecting that the impedance-measuring device 112 is within the vicinity of the defibrillator 106. For example, the defibrillator 106 may shift the first signal 110 from the first frequency to a third frequency based on detecting the impedance-measuring device 112. In some cases, the defibrillator 106 identifies the second frequency of the second signal 114 by detecting the second signal 114 and/or based on the vicinity indicator 118. If the defibrillator 106 determines that the first frequency of the first signal 110 overlaps with, or is within a threshold range of, the second frequency, the defibrillator 106 is configured to shift the first signal 110 from the first frequency to the third frequency, wherein the third frequency is outside of the threshold range of the second frequency. In various cases, the third frequency is in a range of 5 Hz to 1 MHz. In some cases, the defibrillator 106 and/or the impedance-measuring device 112 are configured to shift one or both of the respective carrier frequencies of the first signal 110 and/or the second signal 114 to avoid overlapping carrier frequencies of the first signal 110 and the second signal 114 during the same time interval. In various cases, the defibrillator 106 and the impedance-measuring device 112 are configured to simultaneously detect electrical impedances of the subject 102 by using impedance interrogation signals with different frequencies.

In some implementations, the defibrillator 106 and/or the impedance-measuring device 112 are configured to add one or more additional carrier frequencies to the first signal 110 and/or the second signal 114, wherein the one or more additional carrier frequencies are nonoverlapping with the first frequency and the second frequency. In some cases, one or more additional carrier frequencies are introduced in response to detecting the presence of the defibrillator 106 or the impedance-measuring device 112 electrically coupled to the subject 102. In some examples, the one or more additional carrier frequencies are introduced in response to a selection by the rescuer 104, such as a selection of a multi-shock mode (e.g., a DSD mode) of the defibrillator 106. In various cases, if the first signal 110 includes multiple carrier frequencies, the carrier frequency of the second signal 114 is less likely to overlap the multiple carrier frequencies than it is to overlap a signal carrier frequency (e.g., the first frequency). Accordingly, the addition of multiple carrier frequencies in the first signal 110 or the second signal 114 can result in the defibrillator 105 and/or impedance-measuring device 112 correctly identifying the impedance of the subject 102 by analyzing the amplitude of the voltage or current of a component of the first signal 110 or the second signal 114 corresponding to one of the multiple carrier frequencies.

In some implementations, the defibrillator 106 and/or the impedance-measuring device 112 is configured to remove interference caused by electrical signals output by the other device. For instance, the defibrillator 106 is configured to remove, from the first signal 110, interference caused by the second signal 114. In some examples, the impedance-measuring device 112 is configured to remove, from the second signal 114, interference caused by the first signal 110. According to various implementations, the defibrillator 106 is configured to filter an artifact caused by the second signal 114 from the first signal 110. For instance, the defibrillator 106 is configured to apply, to data representing the first signal 110, a digital filter that substantially rejects one or more carrier frequencies of the second signal 114. In various cases, the defibrillator 106 is configured to multiply the filter with the data if the data is in the frequency domain, or is configured to convolve the filter with the data if the data is in the time domain. In some implementations, the defibrillator 106 applies the filter to each of multiple segments of the data (which respectively correspond to overlapping and/or distinct time intervals) as the defibrillator 106 detects the first signal 110 in real time. Accordingly, the defibrillator 106 may generate filtered data substantially in real time.

Various filters can be utilized to remove the interference. In various cases, the applied filter includes at least one stop band that overlaps a carrier frequency of the second signal 114. In some cases, the filter includes at least one stop band that overlaps at least a portion of a high frequency spectrum, such as a spectrum of greater than 1 MHz. In various examples, the applied filter includes at least one pass band that overlaps a carrier frequency of the first signal 110. The filter, in various cases, includes at least one pass band that overlaps a relatively low-frequency spectrum corresponding to motion artifacts and/or treatment artifacts, such as a pass band that overlaps at least a portion of a frequency spectrum in a range of 0 Hz to 5 Hz.

In some examples, the defibrillator 106 is configured to generate a filter that selectively removes the carrier frequency of the second signal 114 from the first signal 110. For instance, the defibrillator 106 may detect the carrier frequency of the second signal 114 by determining that a frequency domain representation of the first signal 110 includes an amplitude that is greater than a threshold amplitude at a frequency that is distinct from the carrier frequency of the first signal 110. The defibrillator 106, for instance, may infer that the frequency corresponds to the carrier frequency of the second signal 114. According to some cases, the defibrillator 106 may generate a high-Q filter (e.g., a notch filter) with a stop band that specifically overlaps the carrier frequency of the second signal 114, and may apply the generated filter to data representing the first signal 110. For example, the high-Q filter may have a bandwidth that is in a range of 10 MHz to 1 kHz.

In some implementations, the defibrillator 106 is configured to apply a predetermined filter to the first signal 110 to remove interference from the second signal 114. In various cases, the predetermined filter includes a first pass band that overlaps the first frequency (e.g., the carrier frequency) of the first signal 110. The first pass band can be relatively narrow, and may have a bandwidth that is in a range of 1 MHz to 1 kHz, in some cases. In various cases, the predetermined filter includes a second pass band that overlaps relatively low frequencies associated with treatment artifacts (e.g., periodic chest compressions, respiration, or ventilation associated with the subject 102) and/or motion artifact. For example, the second pass band extends from 0 Hz to 5 Hz, according to some cases. In various implementations, the predetermined filter includes a third pass band that corresponds to data modulated into the first signal 110 and/or the second signal 114. In various cases, the predetermined filter includes one or more stop bands that overlap frequencies corresponding to possible impedance interrogation signals that are transmitted by devices other than the defibrillator 106. For example, the stop band(s) may overlap frequencies in a range of 1 kHz to 1 MHz, while excluding the carrier frequency of the first signal 110. In some cases, the defibrillator 106 automatically applies the predetermined filter to data representing the first signal 110. In some examples, the defibrillator 106 selectively applies the predetermined filter to data representing the first signal 110 in response to detecting that the impedance-measuring device 112 is in the vicinity of the defibrillator 106 and/or in response to detecting an input signal from a rescuer 104.

Although the foregoing description focuses on filtering performed by the defibrillator 106, implementations are not so limited. The impedance-measuring device 112, for instance, may be configured to remove an artifact caused by the first signal 110 from the second signal 114 (e.g., from data representing the second signal 114) using similar techniques to those described above.

According to some examples, the defibrillator 106 and the impedance-measuring device 112 are configured to communicate with each other via the first signal 110 and the second signal 114, and/or via another communication interface(s) (e.g., a wired interface(s), a wireless interface(s), or a combination thereof). As described above, in various implementations, the defibrillator 106 is configured to detect the second signal 114 via the first electrode pads 108. In some examples, the impedance-measuring device 112 is configured to detect the first signal 110 via the second electrode pads 116. According to some examples, the defibrillator 106 is configured to encode first data in the first signal 110. For instance, the carrier frequency of the first signal 110 is modulated with a message signal encoding the first data. In various implementations, the message signal is frequency- or phase-modulated into the carrier signal, thereby enabling the impedance-measuring device 112 to differentiate between changes in the impedance of the subject 102 (such as due to the application of chest compressions or other treatment artifact, which can manifest as amplitude modulation of the detected first signal 110) and the encoded first data. The impedance-measuring device 112, for instance, identifies the first data by analyzing the first signal 110. In various cases, the impedance-measuring device 112 is configured to encode second data in the second signal 114. For example, the carrier frequency of the second signal 114 is modulated with a message signal encoding the second data. The defibrillator 106, for example, is configured to identify the second data by analyzing the second signal 114. The first data and/or the second data includes, in various cases, one or more instructions, reports, or other messages. For example, the second signal 114 encodes the vicinity indicator 118 in some cases. In some cases, the second signal 114 and/or the vicinity indicator 118 further includes additional information captured by the impedance-measuring device 112. For example, the second signal 114 and/or the vicinity indicator 118 indicates an impedance captured between the second electrode pads 116, an ECG indicative of an electrical signal detected by the second electrode pads 116 (e.g., an ECG with a chest compression- or ventilation-induced artifact that has been removed due to an analysis of the impedance), a result of a diagnostic algorithm (e.g., a determination by the impedance-measuring device 112 that the subject 102 has a condition, such as VF, that is treatable by the administration of an electrical shock), or any combination thereof.

According to some examples, communication between the defibrillator 106 and the impedance-measuring device 112 is scheduled according to one or more communication windows. In some cases, the defibrillator 106 or the impedance-measuring device 112 defines a first communication window and/or a second communication window. The scheduling device may transmit, to the other device, an indication of the scheduled communication window(s). In some examples, a first device transmits, to a second device, a request for a communication window. For instance, the request may indicate a type of the requesting device, a proposed duration of the communication window, an indication that a multi-shock therapy is indicated for the subject 102, a time at which the request was generated and/or transmitted, or any combination thereof. The receiving device may determine a start time, an end time, a duration, or any combination thereof, of a communication window based on the request. The receiving device may transmit an indication of the scheduled communication window back to the requesting device. In some cases, the request and/or the indication of the communication window are transmitted over a communication interface (e.g., a wireless and/or wired communication interface). In some implementations, one device (e.g., the defibrillator 106) may identify at least one communication window without receiving a request from the other device (e.g., the impedance-measuring device), may communicate the communication window(s) to the other device, and both devices may subsequently communicate over the same channel using the scheduled communication window(s).

Each communication window, for instance, corresponds to a time period that may be scheduled in advance. In various implementations, the requesting device transmits a communication signal (e.g., the first signal 110) during the scheduled communication window. In some examples, the receiving device refrains from transmitting a communication signal (e.g., the second signal 114 or any other impedance interrogation signal) during the communication window. The devices may be configured to transmit communication signals within respective communication windows. During the first communication window, for instance, the defibrillator 106 may transmit the first signal 110 (e.g., encoded with the first data), and the impedance-measuring device 112 may suppress (e.g., refrain from transmitting) the second signal 114. During the second communication window, in some cases, the impedance-measuring device 112 may transmit the second signal 114 (e.g., encoded with the second data), and the defibrillator 106 may suppress (e.g., refrain from transmitting) the first signal 110. Accordingly, interference between the first signal 110 and the second signal 114 can be avoided, even if the defibrillator 106 and the impedance-measuring device 112 are utilizing the same carrier frequency.

In various implementations, the defibrillator 106 and the impedance-measuring device 112 are configured to coordinate a therapy to be administered to the subject 102 based on communications in the first data of the first signal 110 and/or the second data of the second signal 114. In some cases, the defibrillator 106 determines that a sequential-shock therapy is indicated for the subject 102. For example, the defibrillator 106 determines that the ECG of the subject 102 is indicative of refractory VF. In response to determining that the sequential-shock therapy is warranted, the defibrillator 106 encodes, in the first signal 110, an instruction to administer a secondary shock. For example, the instruction indicates a timing of the secondary shock, a timing relationship between a primary shock and the secondary shock, an energy level of the secondary shock, a duration of the secondary shock, a shape of the secondary shock, or any combination thereof. In various cases, the defibrillator 106 is configured to output the primary shock to the subject 102 via the first electrode pads 108. The impedance-measuring device 112, for instance, is configured to output the secondary shock to the subject 102 via the second electrode pads 116, in accordance with the instruction in the first signal 110. In various cases, the primary and secondary shocks are part of a multi-shock therapy, such as a DSD therapy. For instance, the primary and secondary shocks are at least partially temporally overlapping.

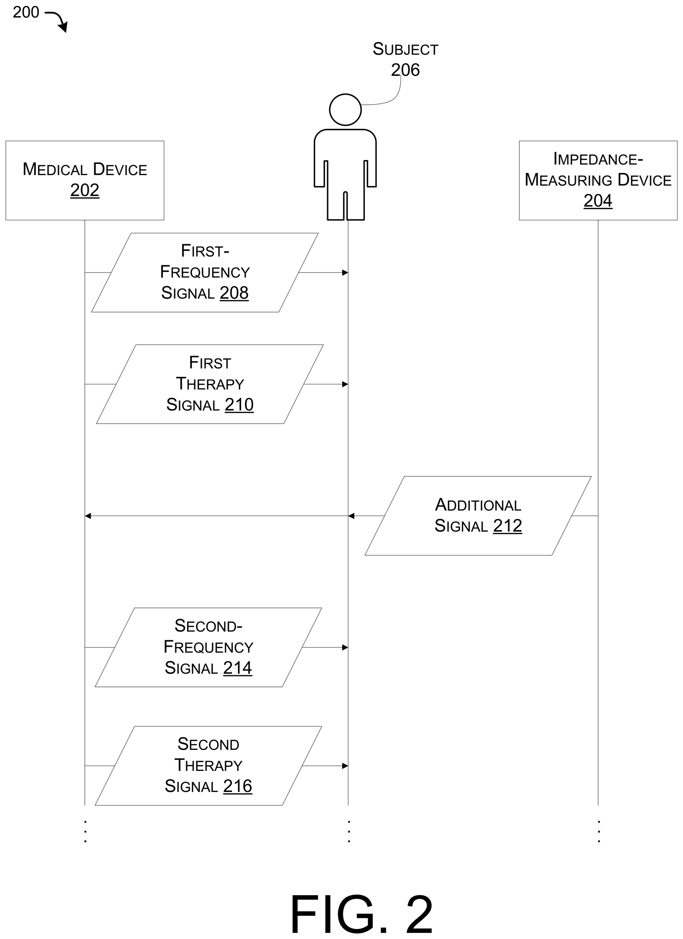

FIG. 2 illustrates example signaling 200 between a medical device 202 and an impedance-measuring device 204 using impedance interrogation signals. Both the medical device 202 and the impedance-measuring device 204 are electrically connected with a subject 206, such as via electrode pads disposed on the skin of the subject 206. In some cases, the medical device 202 is the defibrillator 106, the impedance-measuring device 204 is the impedance-measuring device 112, and the subject 206 is the subject 102.

The medical device 202 is configured to output a first-frequency signal 208 to the subject 206. The first-frequency signal 208, for instance, is an impedance interrogation signal. In various implementations, the medical device 202 includes a measurement circuit configured to output the first-frequency signal 208 at a predetermined RMS current. In various cases, the first-frequency signal 208 is a periodic signal having a first frequency, which is in a range of 5 Hz to 100 kHz. The measurement circuit is configured to detect the voltage necessary to output the first-frequency signal 208 at the predetermined RMS current. The first-frequency signal 208 is output to electrodes disposed on the skin of the subject 206. In some cases, the electrodes are disposed on the chest and/or back of the subject 206. In various cases, the medical device is configured to detect an electrical impedance of an electrical path between the electrodes based on the voltage necessary to output the first-frequency signal 208 at the predetermined RMS current. According to some cases, the electrical impedance is a transthoracic impedance of the subject 206.

In various cases, the medical device 202 is configured to output a first therapy signal 210 to the subject 206. The first therapy signal 210 is a treatment of a medical condition of the subject 206. In some cases, the medical condition includes an arrhythmia, such as VF, VT, or bradycardia. According to some examples, the first therapy signal 210 is output to electrodes disposed on the skin of the subject 206. In some cases, an electrical path between the electrodes intersects the heart of the subject 206.

The first therapy signal 210, for instance, includes an electrical shock, one or more pacing pulses, or some other electrical therapy administered to the subject 206. According to various examples, the medical device 202 selects voltage and/or current of the first therapy signal 210 based, at least in part, on the electrical impedance detected based on the first frequency signal 208. For example, the medical device 202 selects an energy level of the first therapy signal 210 (e.g., in a range of 50 joules (J) to 360 J) and implements a voltage and/or current necessary to achieve the energy level based on a transthoracic impedance of the subject 206.

The impedance-measuring device 204 is configured to output an additional signal 212 to the subject 206. In various implementations, the impedance-measuring device 204 outputs the additional signal 212 to electrodes disposed on the skin of the subject 206. The impedance-measuring device 204 is configured to detect an electrical impedance of an electrical path through the subject 206 and between the electrodes based, at least in part, on the additional signal 212. For instance, the impedance-measuring device 204 is configured to determine a voltage necessary to achieve the additional signal 212 at a predetermined RMS current through the subject 206. According to some cases, the additional signal 212 is a periodic signal with its own frequency. For instance, the frequency of the additional signal 212 is in a range of 5 Hz to 100 kHz. The impedance-measuring device 204 is configured to detect the electrical impedance by determining a voltage level necessary to achieve the additional signal 212 at the predetermined RMS current. The predetermined RMS current, for instance, is in a range of 1 to 50 µA. In some cases, the electrical impedance measured by the impedance-measuring device is a transthoracic impedance of the subject 206.

The additional signal 212, for instance, is also detected by the medical device 202. For example, the medical device 202 detects the additional signal 212 via the same electrodes utilized to output the first-frequency signal 208. According to various cases, the medical device 202 is configured to detect the frequency of the additional signal 212. In some examples, the medical device 202 determines that the first frequency of the first-frequency signal 208 is within a threshold range of the frequency of the additional signal 212. Accordingly, the medical device 202 determines to shift the frequency of its impedance-interrogation signal in order to avoid interference and erroneous impedance measurements due to the additional signal 212.

The medical device 202 is configured to output a second-frequency signal 214 to the subject 206. The second-frequency signal 214, for instance, is an impedance interrogation signal. The second-frequency signal 214 is output at a second frequency, which is different than the first frequency and the frequency of the additional signal 212. For example, the second frequency is outside of a threshold range of the frequency of the additional signal 212. According to some cases, the second frequency is in a range of 5 Hz to 100 kHz. The medical device 202 is configured to measure the electrical impedance of the subject 206 using the second-frequency signal 214 in a manner similar to the techniques utilized to measure the electrical impedance using the first-frequency signal 208.

In some implementations, the medical device 202 and the impedance-measuring device 204 are configured to communicate with one another using impedance interrogation signals. For example, the impedance-measuring device 204 encodes a first message in the additional signal 212 and/or the medical device 202 encodes a second message in the second-frequency signal 214. In some cases, the first message or the second message is an instruction to administer an electrical shock to the subject 206 as part of a multi-shock therapy. For instance, the electrical shock temporally overlaps with another electrical shock output by another device (e.g., the instructing device).

The medical device 202 is configured to output a second therapy signal 216 to the subject 206. The second therapy signal 216 is a treatment of a medical condition of the subject 206. In some cases, the second therapy signal 216 is configured to treat a recurrence of the same medical condition treated by the first therapy signal 210. According to some examples, the second therapy signal 216 is output to electrodes disposed on the skin of the subject 206. In some cases, an electrical path between the electrodes intersects the heart of the subject 206.

The second therapy signal 216, for instance, includes an electrical shock, one or more pacing pulses, or some other electrical therapy administered to the subject 206. According to various examples, the medical device 202 selects voltage and/or current of the second therapy signal 216 based, at least in part, on the electrical impedance detected based on the second-frequency signal 214. For example, the medical device 202 selects an energy level of the second therapy signal 216 and implements a voltage and/or current necessary to achieve the energy level based on the transthoracic impedance of the subject 206. In some cases, the second therapy signal 216 is an electrical shock among multiple electrical shocks in a sequential-shock therapy administered to the subject 206. For instance, the second therapy signal 216 is a primary shock or a secondary shock in a DSD therapy.

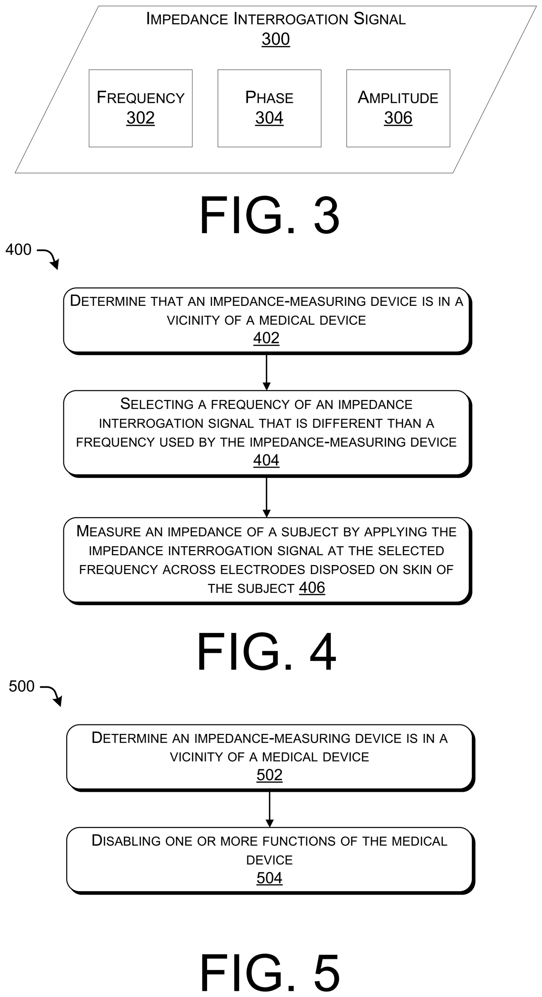

FIG. 3 illustrates an example of an impedance interrogation signal 300. The impedance interrogation signal 300, for example, is an electrical signal having a frequency 302, a phase 304, and an amplitude 306. The frequency 302, phase 304, and amplitude 306 may change over time.

In various cases, the frequency 302 of the impedance interrogation signal is in a range of 5 Hz to 100 kHz. In various cases, the impedance interrogation signal 300 is output, by a medical device, to electrodes disposed on the skin of a subject. The medical device is configured to detect an electrical impedance along a path through the subject and between the electrodes based on the impedance interrogation signal 300. In some cases, the medical device selects the frequency 302 so that the impedance interrogation signal 300 does not interfere with another signal being output to the subject. For example, the frequency 302 is outside of a threshold range of a frequency of another impedance interrogation signal being output to the subject by a separate device at the same time as the impedance interrogation signal 300.

In some cases, the impedance interrogation signal 300 is utilized to output data to the separate device. For example, the medical device modulates the phase 304 and/or the amplitude 306 to encode data that is communicated to the separate device via the impedance interrogation signal 300. In various cases, the medical device amplitude modulation (AM), phase modulation (PM), or phase-amplitude modulation (PAM) on the impedance interrogation signal 300. Various messages can be encoded into the impedance interrogation signal 300 using these techniques. For example, the medical device encodes an instruction to output a treatment signal. In some cases, the treatment signal is an electrical shock that is administered to the subject. For instance, the electrical shock is part of a coordinated, multi-shock therapy, such as a DSD therapy administered jointly by the medical device and the separate device. In some cases, the instruction includes a timing relationship or other timing information associated with the electrical shocks in the coordinated therapy.

FIG. 4 illustrates an example process 400 for adapting a frequency of an impedance interrogation signal based on the presence of an impedance-measuring device. The process 400 is performed by an entity including, for instance, at least one of a medical device, a defibrillator (e.g., a monitor-defibrillator or AED), an impedance-interrogation device, a patient monitor, at least one processor, or a computing device.

At 402, the entity determines that an impedance-measuring device is electrically connected to a subject and/or in a vicinity of a medical device. In some cases, the entity identifies the vicinity of the impedance-measuring device by receiving an input signal from a user. For instance, the input signal indicates selection of a multi-shock mode (e.g., a DSD mode) of the medical device. In some examples, the entity recognizes the vicinity of the impedance-measuring device by receiving a communication signal (e.g., a wireless communication signal) from the impedance-measuring device. In some aspects, the entity recognizes the vicinity of the impedance-measuring device by detecting, from the subject, an electrical signal originating from the impedance-measuring device. For example, the medical device detects an electrical signal output by the impedance-measuring device using the subject's body as a communication medium.

At 404, the entity selects a frequency of an impedance interrogation signal that is different than a frequency used by the impedance-measuring device. The entity, for example, analyzes a waveform representative of the electrical signal output by the impedance-measuring device during a time interval in which the impedance interrogation signal is not output to the subject.

In various cases, the frequency of the impedance interrogation signal is outside of a threshold range of the frequency of an electrical signal output by the impedance-measuring device. The threshold range, for example, is ±100 Hz, ±1 kHz, ±10 kHz, or the like. In some examples, the entity changes the frequency of the impedance interrogation signal based on the frequency used by the impedance-measuring device. For example, if a present frequency of the impedance interrogation signal is within the threshold range of the frequency of the electrical signal output by the impedance-measuring device, then the entity may change the frequency of the impedance interrogation signal. In some examples, the frequency of the impedance interrogation signal is selected from among a set of predetermined frequencies.

In some examples, the entity is configured to negotiate with the impedance-measuring device. For instance, the entity may transmit and/or receive communication signals with the impedance-measuring device indicating the frequency of the electrical signal output by the impedance-measuring device, the frequency of the impedance interrogation signal, or both. One or both of the entity and the impedance-measuring device is configured to adapt the frequency of the signal it outputs based on the communication signals, for instance, to avoid the transmission of overlapping frequencies. In some implementations, the communication signals are transmitted via one or more wireless communication protocols. In some examples, the communication signals indicate a manufacturer of the impedance-measuring device. The entity, for instance, identifies that the manufacturer of the impedance-measuring device is associated with a predetermined frequency, and accordingly selects a different frequency for the impedance interrogation signal in response to identifying the manufacturer.

At 406, the entity measures an impedance of a subject by applying the impedance interrogation signal at the selected frequency across electrodes disposed on skin of the subject. For example, the entity applies a predetermined RMS current through the body of the subject using the impedance interrogation signal. The entity measures the voltage necessary to output the impedance interrogation signal at the predetermined RMS current. Based on the voltage and the predetermined RMS current, the entity identifies the impedance of the subject. In some cases, the impedance is a transthoracic impedance.

FIG. 5 illustrates an example process 500 for disabling functions of a medical device based on the presence of an impedance-measuring device. The process 500 is performed by an entity including, for instance, at least one of a medical device, a defibrillator (e.g., a monitor-defibrillator or AED), an impedance-interrogation device, a patient monitor, at least one processor, or a computing device.

At 502, the entity determines that the impedance-measuring device is electrically connected to a subject and/or is in a vicinity of the medical device. The entity, for example, analyzes a waveform representative of the electrical signal output by the impedance-measuring device during a time interval in which the impedance interrogation signal is not output to the subject.

In various implementations, the entity identifies (e.g., receives) data representing a characteristic of a first interrogation signal transmitted through the subject. For instance, the entity may transmit the first interrogation signal into the subject, and may vary a first characteristic (e.g., a current) of the first interrogation signal over time in accordance with a first carrier frequency. In some cases, the entity generates the data by detecting a second characteristic (e.g., a voltage) of the first interrogation signal over time. The entity may detect the impedance-measuring device by determining that the data includes an artifact associated with a second interrogation signal transmitted through the subject by the impedance-measuring device. The second interrogation signal, for instance, has a second carrier frequency that is different than the first carrier frequency. The impedance-measuring device can be detected, for instance, by detecting the artifact associated with the second carrier frequency in the data representing the characteristic of the first interrogation signal. In some examples, the entity is configured to remove the artifact.

In some aspects, the entity may identify a type of the impedance-measuring device based on the second carrier frequency. For instance, particular makes and models of AEDs (or other impedance-measuring devices) may transmit impedance interrogation signals with particular carrier frequencies. The entity, in various cases, may identify the second carrier frequency and identify the type of the impedance-measuring device based on the second carrier frequency by referencing an entry of a look-up table or other prestored data object, wherein the entry indicates the type. The entity may output a notification indicating the type of the second impedance-measuring device, for instance. In some implementations, the entry of the look-up table further indicates a pairing technique (e.g., whether the impedance-measuring device is compatible with BLUETOOTH^{™}, etc.) that the entity may subsequently utilize to exchange information with the impedance-measuring device.

In some cases, the entity determines that there is a risk to the entity, to the impedance-measuring device, or to the subject based on the type of the impedance-measuring device. For instance, the entry of the look-up table further indicates that the impedance-measuring device is not designed to work in a coordinated fashion with the entity, and may therefore malfunction due to the presence of the entity (e.g., if the entity is electrically coupled with the subject) and/or the presence of the first interrogation signal. Such a malfunction may cause harm to hardware of the entity and/or the impedance-measuring device, or may even cause harm to the subject. For instance, the impedance-measuring device may malfunction by recommending, or administering, an electrical shock to the subject without the subject experiencing a shockable arrhythmia (e.g., VF or VT). In various implementations, the entity may output a warning or other notification indicating the risk.

In some examples, the entity stores, in a record, an indication that the impedance-measuring device was detected in the vicinity of the medical device. The record, for instance, indicates the time at which the impedance-measuring device was detected.

At 504, the entity disables one or more functions of the medical device. In some cases, the entity is configured to output an impedance interrogation signal to the subject. For example, the entity disables the function(s) in response to determining that a frequency of the impedance interrogation signal is within a threshold range of the frequency of the electrical signal output by the impedance-measuring device.

Various functions are disabled by the entity, for instance. In some cases, the entity disables an impedance-measuring function in response to detecting the impedance-measuring device. For example, the entity suppresses the first interrogation signal by at least temporarily refraining from outputting the first interrogation signal to the subject. According to some examples, the entity suppresses the first interrogation signal during a communication window, which may be scheduled in advance (e.g., with or by the impedance-measuring device). In some examples, the entity disables a function of outputting one or more recommendations to administer (or to refrain from administering) one or more electrical shocks to the subject. In some cases, the entity disables a function of detecting motion and/or treatments of the subject. In various implementations, the entity disables pre-shock impedance measurement functions, motion detection, leads-on checks (e.g., a function of determining whether electrodes are attached to the subject), detecting chest compressions, detecting assisted ventilation cycles, detecting for spontaneous respiration, or any combination thereof. In some cases, the entity selectively suppresses the first interrogation signal until the entity performs an impedance-dependent function, such as in a time interval immediately prior to administration of an electrical shock to the subject.

In some examples, the entity disables a function of implementing voltage compensation of one or more defibrillation waveforms. For instance, when the voltage compensation function is enabled, the entity may customize a voltage or current of an electrical shock to be delivered to the subject based on a transthoracic impedance of the subject. The transthoracic impedance, for instance, may be calculated based on the data representing the characteristic of the first interrogation signal. When the impedance-measuring device is detected, however, the entity may instead automatically set a subsequent electrical shock to a predetermined voltage or current.

In some examples, the disabled functions are informed by the impedance-measuring device. For example, the entity may perform voltage compensation, but may perform voltage compensation based on the impedance measurements communicated to the entity by the impedance-measuring device.

In some implementations, the entity automatically transitions to a multi-shock mode (e.g., a DSD mode) in response to detecting the impedance-measuring device. For instance, the entity may refrain from performing monitoring one or more physiological parameters of the subject in the multi-shock mode. In some cases, the entity disables one or more input devices. For instance, the entity may black out a display or disable one or more buttons. In various cases, the entity may output a notification of the multi-shock mode, such as a pop-up notification on the display. When the entity is in the multi-shock mode, in various cases, the entity may function to output a secondary electrical shock in response to detecting a primary electrical shock (e.g., output by the impedance-measuring device), such as from electrodes disposed on the subject. In some examples, the entity may output the secondary electrical shock in response to receiving an instruction from the impedance-measuring device.

In various cases, the entity stores and/or outputs an alert or notification indicating the presence of the impedance-measuring device in the vicinity of the medical device. The alert, for instance, indicates that the impedance-measuring device may cause interference with measurements of the impedance of the subject. In some aspects, the alert indicating that the impedance-measuring device may potentially malfunction. In various cases, the entity outputs a prompt to a user. In response to the prompt, the user may enter an input signal that causes the entity to disable the function(s). Optionally, the entity outputs an alert indicating that the function(s) have been disabled. In various implementations, the entity outputs alerts, notifications, or other information to a user via a display integrated with the entity or a display of an external device communicatively coupled with the entity. Examples of external devices include computing devices, tablet computers, smart phones, virtual reality devices (e.g., VR headsets), augmented reality devices (e.g., AR glasses), or the like. In some cases, the entity stores an indication of the presence of the impedance-measuring device, which can be analyzed during post-event review.

Optionally, the entity initiates a self-check in response to detecting the presence of the impedance-measuring device. For example, the entity may engage in a self-test and associated analysis (e.g., an automated analysis detecting faults within circuitry of the entity itself) when the entity is powered down subsequent to detecting the impedance-measuring device. Accordingly, if the impedance-measuring device damages the entity during use, such damage can be identified and rectified rapidly.

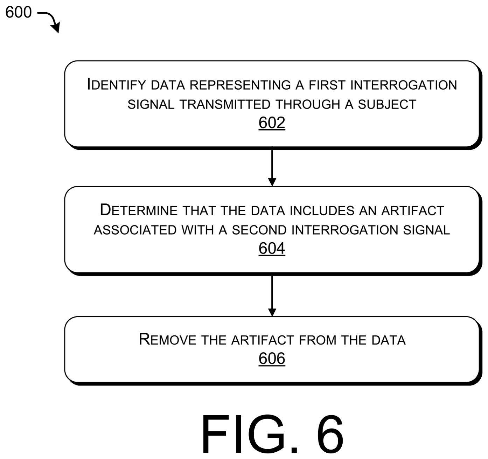

FIG. 6 illustrates an example process 600 for removing, from data representing a first interrogation signal, an artifact caused by a second interrogation signal. The process 600 is performed by an entity including, for instance, at least one of a medical device, a defibrillator (e.g., a monitor-defibrillator or AED), an impedance-interrogation device, a patient monitor, at least one processor, or a computing device.

At 602, the entity identifies data representing a first interrogation signal transmitted through a subject. In various cases, the first interrogation signal has a first carrier frequency. The first carrier frequency may be in a range of 10 Hz to 20 GHz, such as in a range of 1 kHz to 5 MHz.

In some implementations, the entity generates the data by detecting a characteristic (e.g., a current or voltage) of the first interrogation signal. Optionally, the entity transmits at least a component of the first interrogation signal into the subject by controlling another characteristic (e.g., the voltage or current) in accordance with the first carrier frequency. In various implementations, the entity is electrically coupled to electrodes configured to detect and/or transmit the first interrogation signal. For instance, the electrodes are disposed on the torso (e.g., the chest and/or back) of the subject.

In some implementations, the entity demodulates the data. For example, the entity may demodulate the data using data representing a carrier signal of the first interrogation signal, wherein the carrier signal has the first carrier frequency.

At 604, the entity determines that the data includes an artifact associated with a second interrogation signal. In various implementations, the second interrogation signal has a second carrier frequency. The second carrier frequency may be in a range of 10 Hz to 20 GHz, such as in a range of 1 kHz to 5 MHz.

In some cases, the entity detects that an impedance-detecting device is in the vicinity of the entity using any of various relevant techniques described elsewhere herein. In some examples, the entity converts the data from the time domain into the frequency domain (e.g., by performing a Fourier transform, such as a DFT, FFT, or the like, on the data). The entity may infer that the artifact is present if the frequency domain representation of the data includes an amplitude that is greater than a threshold amplitude.

According to some examples, an absolute value of a difference between the first carrier frequency and the second carrier frequency is below a threshold, such that the second carrier frequency interferes with the first carrier frequency, producing a beat artifact in the data. In some cases, when the data is demodulated using the first carrier frequency, the frequency domain representation of the demodulated data includes at least one artifact resembling a sinc function and/or a local peak surrounded by two symmetric lobes. In various cases, the entity is configured to detect the presence of this characteristic artifact.

At 606, the entity removes the artifact from the data. In various examples, the entity applies one or more filters to the data (e.g., prior to demodulation). In some cases, the filter is convolved with the data in the time domain or the frequency domain. In some implementations, the filter is multiplied with the data in the time domain or the frequency domain. In some examples, the effect of the second carrier frequency is a convolution in the frequency domain with a kernel representing the effect of spectral line splitting, such that the artifact can be suppressed by multiplying an inverse of the kernel in the time domain.

In various cases, the filter includes one or more pass bands and one or more stop bands. For example, the pass band(s) include the first carrier frequency and/or relatively low frequencies (e.g., frequencies in a range of 0 to 10 Hz or 0 to 1 kHz). The pass band(s), for instance, avoid suppression of the message signal in the data. In some examples, the stop band(s) include the second carrier frequency and/or any additional artifact caused by the presence of the second carrier frequency. In some implementations, the stop band(s) include frequencies other than the first carrier frequency that are in a possible carrier frequency spectrum (e.g., a spectrum in a range of 10 Hz to 20 GHz, such as in a range of 1 kHz to 5 MHz). According to some cases, the filter includes a notch filter configured to reject the second carrier frequency.

Optionally, the entity performs additional actions in response to removing the artifact from the data. For instance, in some examples, the entity determines an impedance (e.g., a transthoracic impedance) of at least a portion of the subject by analyzing the filtered data. In various cases, the entity infers the impedance by analyzing the amplitude of the data representing the first interrogation signal. Impedance, in various cases, represents current divided by voltage. If the filtered data represents the voltage of the first interrogation signal over time, sans the artifact caused by the second interrogation signal, then the entity may recover the impedance by dividing data representing the current of the first interrogation signal by the filtered data. In some implementations, the entity is configured to determine whether the subject is moving (e.g., spontaneously moving or is being moved, such as by a rescuer) by detecting a motion artifact (e.g., a change) in the impedance of the subject over time. According to some cases, the entity is configured to identify a treatment artifact in the

In some implementations, the entity causes alteration of the first interrogation signal to enhance removal of the artifact caused by the second interrogation signal. For instance, the entity may add a third carrier frequency to the first interrogation signal transmitted into the subject. In some cases, the entity shifts the carrier frequency of the transmitted first interrogation signal from the first carrier frequency to the third carrier frequency. In various implementations, an absolute value of a difference between the third carrier frequency and the second carrier frequency is greater than an absolute value of a difference between the first carrier frequency and the third carrier frequency.

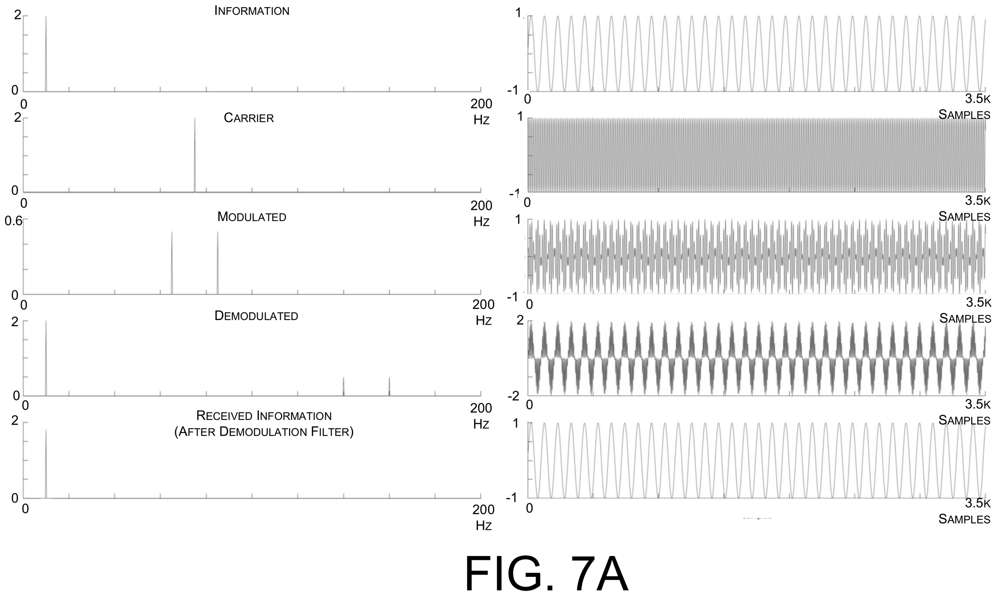

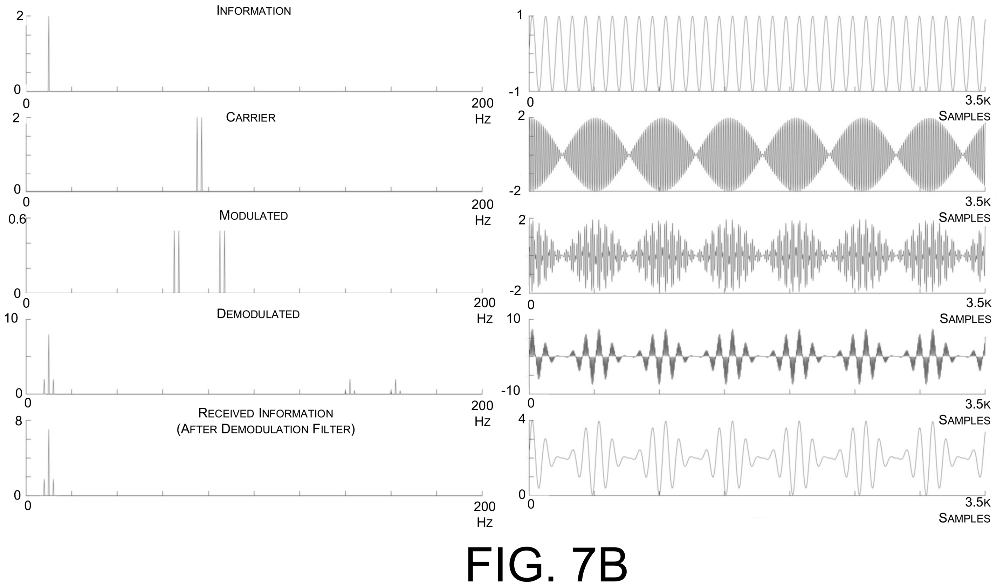

FIGS. 7A and 7B illustrate examples of simulated impedance signals. The frequencies and times portrayed by FIGS. 7A and 7B are not necessarily drawn to scale. FIG. 7A illustrates an example of data representing processing of an impedance interrogation signal with a single carrier wave that is modulated with information. The left column of FIG. 7A portrays the frequency domain representations of various elements of the impedance interrogation signal. The right column of FIG 7A portrays time domain representations of various elements of the impedance interrogation signal.

The first (uppermost) row of FIG. 7A represents a message signal encoded into the impedance interrogation signal. In this example, the information of the message signal is represented by a single-frequency sine wave in the time domain. In some cases, the information represented by the message signal includes a communication that can be detected by an external device. For instance, a medical device outputting the impedance interrogation signal of FIG. 7A may communicate with another medical device using the message signal. In some cases, the message signal represents a treatment being administered to the subject. For example, the message signal may represent chest compressions administered to the subject, ventilation administered to the subject, or respiration (e.g., spontaneous breathing) of the subject. In some implementations, the message signal represents motion of the body of the subject, such as during transport (e.g., in an ambulance or using a mobile cot) or the like.

The second row of FIG. 7A represents the carrier signal of the impedance interrogation signal. In this example, the carrier signal is also represented by a single-frequency sine wave in the time domain. However, the frequency of the carrier signal is higher than the frequency of the information signal. For instance, in various implementations, the carrier signal has a frequency in a range of 10 Hz to 20 GHz (e.g., 1 kHz to 5 MHz). In various implementations, a device may transmit an electrical signal into a subject (e.g., a patient), wherein an amplitude of a characteristic of the electrical signal (e.g., a voltage or current) varies over time in accordance with the varying amplitude of the carrier signal.

The third row of FIG. 7A represents a modulated signal. In particular, the modulated signal of FIG. 7A is generated by performing amplitude modulation. The modulated signal is generated by changing the amplitude of the carrier signal according to the amplitude of the message signal. For instance, the carrier signal and the message signal are multiplied. In the frequency domain, the modulated signal has two sidebands: a lower sideband representing a difference between the frequencies of the message signal and the carrier signal, and an upper sideband representing a sum of the frequencies of the message signal and the carrier signal. According to various implementations, a device detects a characteristic of the electrical signal (e.g., the current or voltage) that has been transmitted into the subject. The device, for instance, can be the same device that transmitted the electrical signal. In various cases, the amplitude of the detected characteristic of the electrical signal may vary in accordance with the varying amplitude of the modulated signal. In various cases, the device may detect an electrical impedance (e.g., a transthoracic impedance) of the subject based on the transmitted and detected characteristics of the electrical signal. In some cases, the device transmitting the impedance interrogation signal may transmit it with a characteristic that varies over time consistently with the modulated signal. Although FIG. 7A illustrates an example of amplitude modulation, implementations of the present disclosure are not so limited. In some cases, an impedance interrogation signal can encode data using frequency modulation and/or phase modulation.

The fourth row of FIG. 7A represents a demodulated signal. For instance, data representing the demodulated signal may be generated by demodulating the modulated signal of the third row. Amplitude demodulation, for instance, can be performed by rectifying and filtering the modulated signal. In some implementations, synchronous detection is performed on the modulated signal to generate the demodulated signal. In various cases, the demodulated signal may have a band in the frequency domain that is consistent with the information signal of the first row and another band in the frequency domain that is consistent with the carrier signal of the second row. In various cases, the demodulation is performed based on the known carrier signal. For instance, the demodulated signal is generated by mixing the modulated signal with a replica of the carrier signal.

The fifth row of FIG. 7A represents received (e.g., extracted) information from the detected characteristic of the impedance interrogation signal. In various cases, the received information has a band in the frequency domain that is consistent with the band of the information signal portrayed in the first row. In various implementations, the received information signal is generated by applying a filter to the modulated signal or the demodulated signal, wherein the filter rejects a frequency of the carrier signal.

In various implementations, the signals of FIG. 7A can be utilized by a first device (e.g., a medical device) detecting the impedance of a subject. However, if there is a second device that is connected with the subject, and that second device is transmitting another impedance interrogation signal into the subject, then the impedance interrogation signal detected by the first device may have different characteristics.

FIG. 7B illustrates an example of data representing processing of an impedance interrogation signal with multiple carrier waves. The left column of FIG. 7B portrays the frequency domain representations of various elements of the impedance interrogation signal. The right column of FIG 7B portrays time domain representations of various elements of the impedance interrogation signal.

The information signal portrayed in FIG. 7B is identical to that of FIG. 7A. However, the carrier signal in the second row of FIG. 7B includes two bands respectively representing two different carrier frequencies. For instance, one of those bands may represent the carrier signal of a first impedance interrogation signal transmitted by a first device into the subject, and the other band may represent the carrier signal of a second impedance interrogation signal transmitted by a second device into the subject. A device detecting the first impedance interrogation signal, for instance, may detect a signal with two carrier frequencies corresponding to the first and the second impedance interrogation signals. In other words, the carrier signal has a first carrier frequency and a second carrier frequency.

In various cases, the modulated signal of FIG. 7B includes four frequency domain peaks. In the frequency domain, the modulated signal has two lower sidebands representing a differences between the frequencies of the message signal and the respective first and second frequencies of the carrier signal; and two upper sidebands representing sums of the frequency of the message signal and the respective first and second frequencies of the carrier signal.

In various cases, a device detecting an impedance interrogation signal with a characteristic that varies over time in accordance with the modulated signal of FIG. 7B may generate the demodulated signal based on one of the carrier frequencies, such as the first carrier frequency. For example, synchronous detection can be performed by mixing a carrier signal representing only the first carrier frequency with the modulated signal. However, the presence of the second carrier frequency yields an artifact in the demodulated signal. In particular cases, the artifact is in the form of sidebands surrounding the band representing the frequency of the information signal in the demodulated signal. In other words, the artifact manifests as a splitting of the sideband corresponding to the frequency of the information signal into three distinct bands. The second carrier frequency yields a beat artifact in the impedance interrogation signal. Even if the first carrier frequency is filtered from the modulated and/or demodulated signal, the received information signal retains those sidebands, such that the received information signal is distinct from the original information signal. In various implementations, the device is configured to remove a component corresponding to the second carrier frequency. The device, for instance, may apply a filter to data representing the modulated signal and/or data representing the demodulated signal. The filter, for instance, rejects a component of the corresponding signal that is injected by the second impedance interrogation signal. Accordingly, the information signal can be accurately recovered as the received information signal.

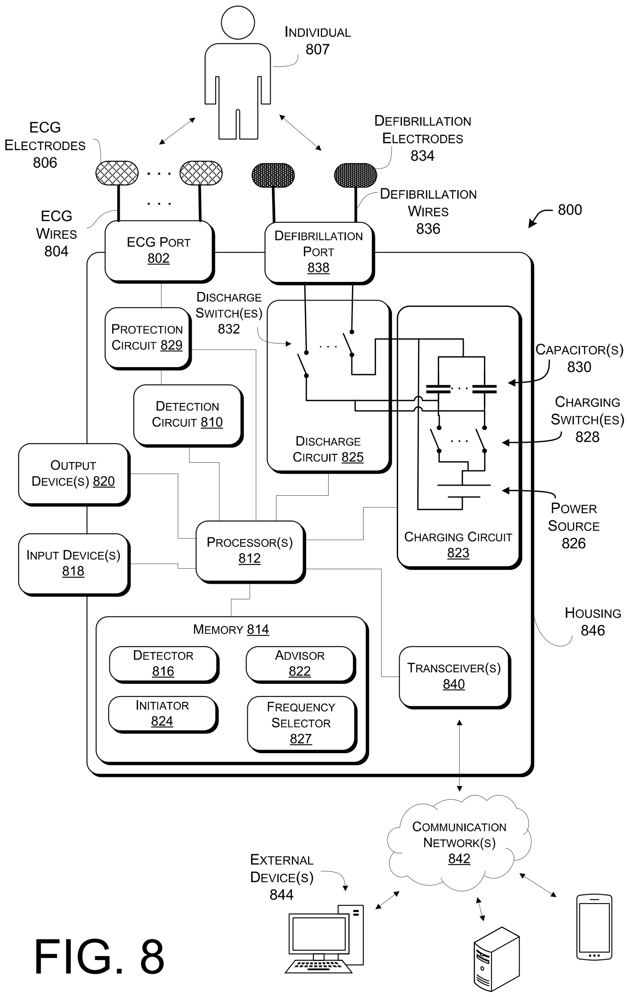

FIG. 8 illustrates an example of an external defibrillator 800 configured to perform various functions described herein. For example, the external defibrillator 800 is the defibrillator 106 or impedance-measuring device 112 described above with reference to FIG. 1.

The external defibrillator 800 includes an ECG port 802 connected to multiple ECG wires 804. In some cases, the ECG wires 804 are removeable from the ECG port 802. For instance, the ECG wires 804 are plugged into the ECG port 802 via connectors. The ECG wires 804 are connected to ECG electrodes 806, respectively. In various implementations, the ECG electrodes 806 are disposed on different locations on an individual 808. A detection circuit 810 is configured to detect relative voltages between the ECG electrodes 806. These voltages are indicative of the electrical activity of the heart of the individual 808.

In various implementations, the ECG electrodes 806 are in contact with the different locations on the skin of the individual 808. In some examples, a first one of the ECG electrodes 806 is placed on the skin between the heart and right arm of the individual 808, a second one of the ECG electrodes 806 is placed on the skin between the heart and left arm of the individual 808, and a third one of the ECG electrodes 806 is placed on the skin between the heart and a leg (either the left leg or the right leg) of the individual 808. In these examples, the detection circuit 810 is configured to measure the relative voltages between the first, second, and third ECG electrodes 806. Respective pairings of the ECG electrodes 806 are referred to as "leads," and the voltages between the pairs of ECG electrodes 806 are known as "lead voltages." In some examples, more than three ECG electrodes 806 are included, such that 8-lead or 12-lead ECG signals are detected by the detection circuit 810.

The detection circuit 810 includes at least one analog circuit, at least one digital circuit, or a combination thereof. The detection circuit 810 receives the analog electrical signals from the ECG electrodes 806, via the ECG port 802 and the ECG wires 804. In some cases, the detection circuit 810 includes one or more analog filters configured to filter noise and/or artifact from the electrical signals. The detection circuit 810 includes an analog-to-digital (ADC) in various examples. The detection circuit 810 generates a digital signal indicative of the analog electrical signals from the ECG electrodes 806. This digital signal can be referred to as an "ECG signal" or an "ECG."

In some cases, the detection circuit 810 further detects an electrical impedance between at least one pair of the ECG electrodes 806. For example, the detection circuit 810 includes, or otherwise controls, a power source that applies a known current (or voltage) across a pair of the ECG electrodes 806 and detects a resultant voltage (or current) between the pair of the ECG electrodes 806. The impedance is generated based on the applied signal (voltage or current) and the resultant signal (current or voltage). In some cases, the detection circuit 810 detects the impedance via impedance matching. In various cases, the impedance corresponds to respiration of the individual 808, chest compressions performed on the individual 808, and other physiological states of the individual 808. In various examples, the detection circuit 810 includes one or more analog filters configured to filter noise and/or artifact from the resultant signal. The detection circuit 810 generates a digital signal indicative of the impedance using an ADC. This digital signal can be referred to as an "impedance signal" or an "impedance." According to some examples, the applied signal has a frequency in a range of 5 Hz to 100 kHz.

The detection circuit 810 provides the ECG signal and/or the impedance signal one or more processors 812 in the external defibrillator 800. In some implementations, the processor(s) 812 includes a central processing unit (CPU), a graphics processing unit (GPU), both CPU and GPU, or other processing unit or component known in the art.

The processor(s) 812 is operably connected to memory 814. In various implementations, the memory 814 is volatile (such as random access memory (RAM)), non-volatile (such as read only memory (ROM), flash memory, etc.) or some combination of the two. The memory 814 stores instructions that, when executed by the processor(s) 812, causes the processor(s) 812 to perform various operations. In various examples, the memory 814 stores methods, threads, processes, applications, objects, modules, any other sort of executable instruction, or a combination thereof. In some cases, the memory 814 stores files, databases, or a combination thereof. In some examples, the memory 814 includes, but is not limited to, RAM, ROM, electrically erasable programmable read-only memory (EEPROM), flash memory, or any other memory technology. In some examples, the memory 814 includes one or more of CD-ROMs, digital versatile discs (DVDs), content-addressable memory (CAM), or other optical storage, magnetic cassettes, magnetic tape, magnetic disk storage or other magnetic storage devices, or any other medium which can be used to store the desired information and which can be accessed by the processor(s) 812 and/or the external defibrillator 800. In some cases, the memory 814 at least temporarily stores the ECG signal and/or the impedance signal.

In various examples, the memory 814 includes a detector 816, which causes the processor(s) 812 to determine, based on the ECG signal and/or the impedance signal, whether the individual 808 is exhibiting a particular heart rhythm. For instance, the processor(s) 812 determines whether the individual 808 is experiencing a shockable rhythm that is treatable by defibrillation. Examples of shockable rhythms include VF and VT. In some examples, the processor(s) 812 determines whether any of a variety of different rhythms (e.g., asystole, sinus rhythm, atrial fibrillation (AF), etc.) are present in the ECG signal.

The processor(s) 812 is operably connected to one or more input devices 818 and one or more output devices 820. Collectively, the input device(s) 818 and the output device(s) 820 function as an interface between a user and the defibrillator 800. The input device(s) 818 is configured to receive an input from a user and includes at least one of a keypad, a cursor control, a touch-sensitive display, a voice input device (e.g., a microphone), a haptic feedback device (e.g., a gyroscope), or any combination thereof. The output device(s) 820 includes at least one of a display, a speaker, a haptic output device, a printer, or any combination thereof. In various examples, the processor(s) 812 causes a display among the input device(s) 818 to visually output a waveform of the ECG signal and/or the impedance signal. In some implementations, the input device(s) 818 includes one or more touch sensors, the output device(s) 820 includes a display screen, and the touch sensor(s) are integrated with the display screen. Thus, in some cases, the external defibrillator 800 includes a touchscreen configured to receive user input signal(s) and visually output physiological parameters, such as the ECG signal and/or the impedance signal.

In some examples, the memory 814 includes an advisor 822, which, when executed by the processor(s) 812, causes the processor(s) 812 to generate advice and/or control the output device(s) 820 to output the advice to a user (e.g., a rescuer). In some examples, the processor(s) 812 provides, or causes the output device(s) 820 to provide, an instruction to perform CPR on the individual 808. In some cases, the processor(s) 812 evaluates, based on the ECG signal, the impedance signal, or other physiological parameters, CPR being performed on the individual 808 and causes the output device(s) 820 to provide feedback about the CPR in the instruction. According to some examples, the processor(s) 812, upon identifying that a shockable rhythm is present in the ECG signal, causes the output device(s) 820 to output an instruction and/or recommendation to administer a defibrillation shock to the individual 808.

The memory 814 also includes an initiator 824 which, when executed by the processor(s) 812, causes the processor(s) 812 to control other elements of the external defibrillator 800 in order to administer a defibrillation shock to the individual 808. In some examples, the processor(s) 812 executing the initiator 824 selectively causes the administration of the defibrillation shock based on determining that the individual 808 is exhibiting the shockable rhythm and/or based on an input from a user (received, e.g., by the input device(s) 818. In some cases, the processor(s) 812 causes the defibrillation shock to be output at a particular time, which is determined by the processor(s) 812 based on the ECG signal and/or the impedance signal.

In various cases, the memory 814 further includes a frequency selector 827 that, when executed by the processor(s) 812, causes the processor(s) 812 to select a frequency of the applied signal utilized to measure the impedance of the individual 807. In some cases, the frequency selector 827 chooses the frequency based on a frequency of an electrical signal applied to the individual 807 by another medical device. For instance, the detection circuit 810 is configured to detect the electrical signal applied to the individual 807 by the other medical device. In some examples, the frequency selector 827 deactivates one or more impedance-dependent functions of the defibrillator 800 based, at least in part, on the electrical signal applied to the individual 807 by the other medical device.

The processor(s) 812 is operably connected to a charging circuit 823 and a discharge circuit 825. In various implementations, the charging circuit 823 includes a power source 826, one or more charging switches 828, and one or more capacitors 830. The power source 826 includes, for instance, a battery. The processor(s) 812 initiates a defibrillation shock by causing the power source 826 to charge at least one capacitor among the capacitor(s) 830. For example, the processor(s) 812 activates at least one of the charging switch(es) 828 in the charging circuit 823 to complete a first circuit connecting the power source 826 and the capacitor to be charged. Then, the processor(s) 812 causes the discharge circuit 825 to discharge energy stored in the charged capacitor across a pair of defibrillation electrodes 834, which are in contact with the individual 808. For example, the processor(s) 812 deactivates the charging switch(es) 828 completing the first circuit between the capacitor(s) 830 and the power source 826, and activates one or more discharge switches 832 completing a second circuit connecting the charged capacitor 830 and at least a portion of the individual 808 disposed between defibrillation electrodes 834.

The energy is discharged from the defibrillation electrodes 834 in the form of a defibrillation shock. For example, the defibrillation electrodes 834 are connected to the skin of the individual 808 and located at positions on different sides of the heart of the individual 808, such that the defibrillation shock is applied across the heart of the individual 808. The defibrillation shock, in various examples, depolarizes a significant number of heart cells in a short amount of time. The defibrillation shock, for example, interrupts the propagation of the shockable rhythm (e.g., VF or pulseless VT) through the heart. In some examples, the defibrillation shock is 200 J or greater with a duration of about 0.015 seconds. In some cases, the defibrillation shock has a multiphasic (e.g., biphasic) waveform. The discharge switch(es) 832 are controlled by the processor(s) 812, for example. In various implementations, the defibrillation electrodes 834 are connected to defibrillation leads 836. The defibrillation wires 836 are connected to a defibrillation port 838, in implementations. According to various examples, the defibrillation wires 836 are removable from the defibrillation port 838. For example, the defibrillation wires 836 are plugged into the defibrillation port 838.

In various implementations, the processor(s) 812 is operably connected to one or more transceivers 840 that transmit and/or receive data over one or more communication networks 842. For example, the transceiver(s) 840 includes a network interface card (NIC), a network adapter, a local area network (LAN) adapter, or a physical, virtual, or logical address to connect to the various external devices and/or systems. In various examples, the transceiver(s) 840 includes any sort of wireless transceivers capable of engaging in wireless communication (e.g., radio frequency (RF) communication). For example, the communication network(s) 842 includes one or more wireless networks that include a 3^{rd} Generation Partnership Project (3GPP) network, such as a Long Term Evolution (LTE) radio access network (RAN) (e.g., over one or more LTE bands), a New Radio (NR) RAN (e.g., over one or more NR bands), or a combination thereof. In some cases, the transceiver(s) 840 includes other wireless modems, such as a modem for engaging in WI-FI^{®}, WIGIG^{®}, WIMAX^{®}, BLUETOOTH^{®}, or infrared communication over the communication network(s) 842.

The defibrillator 800 is configured to transmit and/or receive data (e.g., ECG data, impedance data, data indicative of one or more detected heart rhythms of the individual 808, data indicative of one or more defibrillation shocks administered to the individual 808, etc.) with one or more external devices 844 via the communication network(s) 842. The external devices 844 include, for instance, mobile devices (e.g., mobile phones, smart watches, etc.), Internet of Things (IoT) devices, medical devices (e.g., a primary defibrillator, a secondary defibrillator, an impedance-measuring device, etc.), computers (e.g., laptop devices, servers, etc.), or any other type of computing device configured to communicate over the communication network(s) 842. In some examples, the external device(s) 844 is located remotely from the defibrillator 800, such as at a remote clinical environment (e.g., a hospital). According to various implementations, the processor(s) 812 causes the transceiver(s) 840 to transmit data to the external device(s) 844. In some cases, the transceiver(s) 840 receives data from the external device(s) 844 and the transceiver(s) 840 provide the received data to the processor(s) 812 for further analysis. In some cases, the defibrillator 800 is configure to transmit or receive a shock instruction via the communication network(s) 842.

In various implementations, the external defibrillator 800 also includes a housing 846 that at least partially encloses other elements of the external defibrillator 800. For example, the housing 846 encloses the detection circuit 810, the processor(s) 812, the memory 814, the charging circuit 823, the transceiver(s) 840, or any combination thereof. In some cases, the input device(s) 818 and output device(s) 820 extend from an interior space at least partially surrounded by the housing 846 through a wall of the housing 846. In various examples, the housing 846 acts as a barrier to moisture, electrical interference, and/or dust, thereby protecting various components in the external defibrillator 800 from damage.

In some implementations, the external defibrillator 800 is an AED operated by an untrained user (e.g., a bystander, layperson, etc.) and can be operated in an automatic mode. In automatic mode, the processor(s) 812 automatically identifies a rhythm in the ECG signal, makes a decision whether to administer a defibrillation shock, charges the capacitor(s) 830, discharges the capacitor(s) 830, or any combination thereof. In some cases, the processor(s) 812 controls the output device(s) 820 to output (e.g., display) a simplified user interface to the untrained user. For example, the processor(s) 812 refrains from causing the output device(s) 820 to display a waveform of the ECG signal and/or the impedance signal to the untrained user, in order to simplify operation of the external defibrillator 800.

In some examples, the external defibrillator 800 is a monitor-defibrillator utilized by a trained user (e.g., a clinician, an emergency responder, etc.) and can be operated in a manual mode or the automatic mode. When the external defibrillator 800 operates in manual mode, the processor(s) 812 cause the output device(s) 820 to display a variety of information that may be relevant to the trained user, such as waveforms indicating the ECG data and/or impedance data, notifications about detected heart rhythms, and the like.

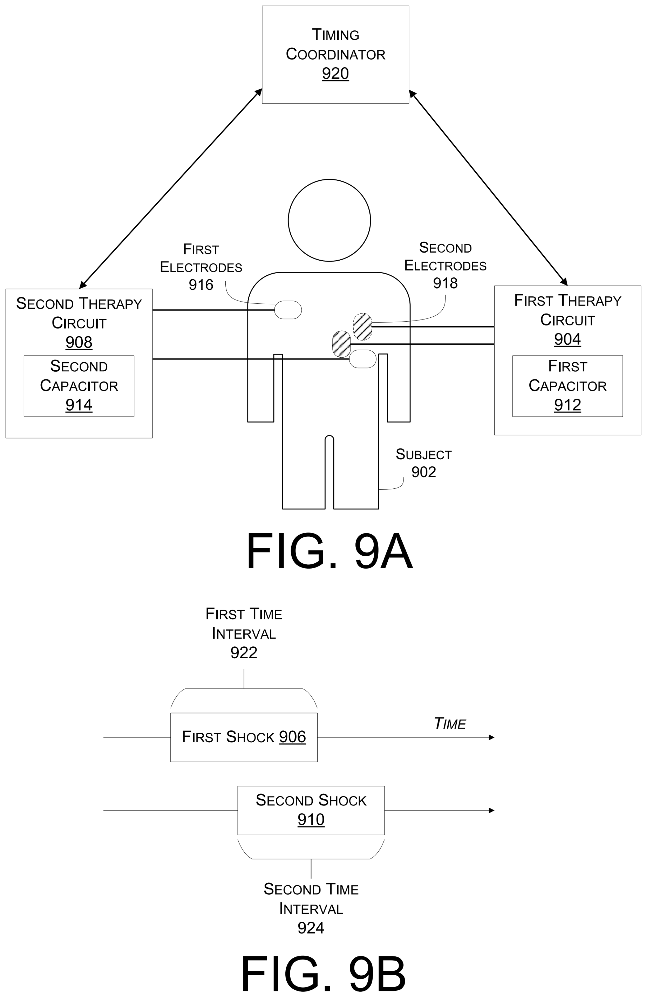

FIGS. 9A and 9B illustrate examples of environments and timing related to administering a multi-shock therapy. FIG. 9A shows an environment configured to administer the multi-shock therapy. FIG. 9B shows a timing relationship of multiple shocks administered in the multi-shock therapy.

In various cases, a subject 902 has a shockable arrhythmia that is treatable by an electrotherapy. For example, the subject 902 may have a shockable cardiac arrhythmia, such as VF or pulseless VT. In some cases, however, conventional, single-shock electrotherapies do not resolve the medical condition. For example, the subject 902 may be experiencing VF that does not resolve after the administration of a single biphasic electrical shock administered by a defibrillator. For instance, the subject 902 may have refractory VF.

In various implementations of the present disclosure, the medical condition of the subject 902 is treatable by administration of a multi-shock therapy. Specifically, a first therapy circuit 904 is configured to output a first shock 906 to the subject 902 and a second therapy circuit 908 is configured to output a second shock 910 to the subject 902. In various cases, the first shock 906 and the second shock 910 temporally overlap in time, at least partially. For example, a start time of the second shock 910 occurs after the start time of the first shock 906, but the start time of the second shock 910 occurs before the end time of the first shock 906. In various cases, the first shock 906 is a biphasic shock and/or the second shock 910 is a biphasic shock. In some cases, the first shock 906 and the second shock 910 are temporally nonoverlapping. In some implementations, the first shock 906 is a monophasic shock and/or the second shock 910 is a biphasic shock. In some cases, the first shock 906 has a shorter duration and/or lower voltage amplitude than the second shock 910.

The first therapy circuit 904 outputs the first shock 906 by discharging a first capacitor 912. Similarly, the second therapy circuit 908 outputs the second shock 910 by discharging a second capacitor 914. In various implementations, one or more power sources are configured to charge the first capacitor 912 and/or the second capacitor 914 prior to discharge. In various cases, the first therapy circuit 904 includes a first H-bridge circuit including the first capacitor 912 and/or the second therapy circuit 908 includes a second H-bridge circuit including the second capacitor 914. The first H-bridge circuit and the second H-bridge circuit are configured to output the first shock 906 and the second shock 910 as biphasic shocks, for instance, via sequential activation of switches in the first H-bridge circuit and the second H-bridge circuit.

The first therapy circuit 904 is configured to output the first shock 906 to first electrodes 916. The second therapy circuit 908 is configured to output the second shock 910 to second electrodes 918. In various cases, the first electrodes 916 and/or the second electrodes 918 are disposed externally on the skin of the subject 902. For example, the first electrodes 916 and/or the second electrodes 918 are adhered to the skin of the subject 902. In various implementations, the first electrodes 916 and the second electrodes 918 are associated with different shock vectors. For instance, a first shock vector extends between the first electrodes 916 and a second shock vector extends between the second electrodes 918, wherein the first shock vector and the second shock vector are different. For example, the first shock vector may be an anterior-lateral position and the second shock vector may be an anterior-posterior position. In various implementations, the first shock vector and the second shock vector both extend through the heart of the subject 902. Although FIG. 9A illustrates the first electrodes 916 as being separate from the second electrodes 918, implementations are not so limited. For example, one electrode may be shared among the first electrodes 916 and the second electrodes 918.

The first therapy circuit 904 and the second therapy circuit 908 are distributed among one or more devices. In some cases, the first therapy circuit 904 is part of a first external defibrillator and the second therapy circuit 908 is part of a second external defibrillator. For example, the first external defibrillator and the second external defibrillator are both monitor-defibrillators, both AEDs, or a monitor-defibrillator and an AED. In some cases, the first therapy circuit 904 is integrated into the defibrillator 1106 and the second therapy circuit 908 is integrated into the impedance-measuring device 112. In some cases, the first therapy circuit 904 or the second therapy circuit 908 is integrated into an accessory device without monitoring capabilities, and which is solely designed to output electrical shocks upon receiving an input signal from a separate device. For example, the accessory device may lack, or be disconnected from, one or more sensors configured to identify one or more physiological parameters of the subject 902. In some cases, the accessory device lacks a display, speaker, or other user interface device. In some implementations, the first therapy circuit 904 and the second therapy circuit 908 are integrated into the same device, such as the same monitor-defibrillator. In some cases, the first therapy circuit 904 and the second therapy circuit 908 are integrated into multiple devices detecting electrical impedances of the subject 902 at the same time.

Optionally, a timing coordinator 920 is configured to cause the first therapy circuit 904 to output the first shock 906 during a first time interval 922 and/or to cause the second therapy circuit 908 to output the second shock 910 during the second time interval 924. In some cases, the first time interval 922 and/or the second time interval 924 begin when another (e.g., predetermined) time interval has expired. For example, the timing coordinator 920 outputs one or more signals (e.g., electrical signals, communication signals, etc.) to the first therapy circuit 904 and/or the second therapy circuit 908. Upon receiving the signal(s) from the timing coordinator 920, the first therapy circuit 904 may discharge the first capacitor 912 during the first time interval 922 and/or the second therapy circuit 908 may discharge the second capacitor 914 during the second time interval 924. The timing coordinator 920 can be implemented in hardware (e.g., a circuit), software (e.g., instructions executed by at least one processor), or a combination thereof. In some cases, the timing coordinator 920 is a standalone device. Examples of standalone timing devices that can serve as the timing coordinator 920 are described in U.S. Pat. No. 10,981,014, which is incorporated by reference herein in its entirety. In some examples, the timing coordinator 920 is integrated into the same device as the first therapy circuit 904 and/or the second therapy circuit 908.

In some cases, the coordinated therapy can be administered even if the first therapy circuit 904 and the second therapy circuit 908 are in separate devices having different manufacturers. For instance, the timing coordinator 920 may be configured to determine that different defibrillators including the first therapy circuit 904 and the second therapy circuit 908 have different manufacturers. For instance, the second therapy circuit 908 may be in a defibrillator that is not capable of, or configured to, communicate with the timing coordinator 920. In some cases, the timing coordinator 920 causes the first therapy circuit 904 to output the first shock 906 in response to detecting the expiration of a time interval associated with a known pattern of discharge associated with the manufacturer of the defibrillator including the second therapy circuit 908. Accordingly, the timing coordinator 920 may nevertheless cause the first shock 906 and the second shock 910 to be output in a coordinated fashion with an optimized therapeutic benefit.

Various timing relationships between the first time interval 922 and the second time interval 924 can be implemented according to various implementations of the present disclosure. In some cases, a delay between the start times (i.e., the leading edges) of the first time interval 922 and the second time interval 924 is in a range of 0 and 250 milliseconds (ms). In some cases, the delay between the start times of the first time interval 922 and the second time interval 924 is in a range of -250 and 0 ms. Although FIG. 9B illustrates the first time interval 922 and the second time interval 924 as having equivalent durations, implementations are not so limited. For example, the first time interval 922 may be longer or shorter than the second time interval 924. In some cases, the first time interval 922 and the second time interval 924 are non-overlapping. Various timing relationships are described in U.S. Pat. No. 10,702,701, which is incorporated by reference herein in its entirety.

In various cases, the timing coordinator 920 is configured to detect the first shock 906 and may cause the second therapy circuit 908 to output the second shock 910 in response. For example, the timing coordinator 920 may detect a signal indicative of the discharge of the first shock 906, and may output a signal that causes the second therapy circuit 908 to discharge the second shock 910. In some examples, the timing coordinator 920 is inductively coupled with the first therapy circuit 904 and/or the first electrodes 916, which enables the timing coordinator 920 to detect the discharge of the first shock 906. Various techniques for detecting the discharge of a first shock in order to cause the application of a second shock in DSD therapy are described in U.S. Pat. Nos. 10,625,088 and 10,632,320, which are incorporated by reference herein in their entirety.

### EXAMPLE CLAUSES

The following example clauses provide various implementations of the present disclosure. However, implementations of the present disclosure are not limited to any of the example clauses presented herein.
1. A medical device, including: a detection circuit configured to: output, to electrodes configured to be disposed on a subject, a first interrogation signal having a first carrier frequency and a first current amplitude; and detect a voltage of the first interrogation signal; a display; and a processor configured to: detect, in data representing the voltage of the first interrogation signal, an artifact associated with a second interrogation signal having a second carrier frequency; remove, from the data representing the voltage of the first interrogation signal, the artifact; in response to removing the artifact, determine, by analyzing the data representing the voltage of the first interrogation signal, a transthoracic impedance of the subject; detect, by analyzing the transthoracic impedance of the subject, chest compressions administered to the subject; and cause the display to output an indication of the chest compressions.
2. The medical device of clause 1, wherein the processor is configured to detect the artifact associated with the second interrogation signal by: converting the data representing the voltage of the first interrogation signal into a frequency domain; generating demodulated data by demodulating the data representing the voltage of the first interrogation signal in the frequency domain; and determining that an amplitude of the demodulated data corresponding to the second carrier frequency is above a threshold, and wherein the processor is configured to remove the artifact by: applying, to the data representing the voltage of the first interrogation signal in the frequency domain, a filter having a pass band that overlaps the first carrier frequency and a stop band that overlaps the second carrier frequency.
3. The medical device of clause 1 or 2, the medical device being a first impedance-measuring device, wherein the processor is further configured to: in response to detecting, in the data representing the voltage of the first interrogation signal, the artifact associated with the second interrogation signal having the second carrier frequency, cause the display to output a notification indicating a presence of a second impedance-measuring device connected to the subject.
4. A method, including: detecting, at a first set of electrodes disposed on a subject, a first interrogation signal having a first carrier frequency; detecting, in data representing the first interrogation signal, an artifact associated with a second interrogation signal having a second carrier frequency; removing, from the data representing first interrogation signal, the artifact; and determining, by analyzing the data representing the first interrogation signal, a transthoracic impedance of the subject.
5. The method of clause 4, wherein detecting, in the data representing the first interrogation signal, the artifact associated with the second interrogation signal includes: converting the data from a time domain to a frequency domain; in response to converting the data from the time domain to the frequency domain, identifying an amplitude of the data corresponding to the second carrier frequency; and determining that the amplitude of the data is greater than a threshold amplitude.
6. The method of clause 4 or 5, wherein the first carrier frequency and the second carrier frequency are in a range of about 500 Hertz (Hz) to about 1 MHz.
7. The method of any of clauses 4 to 6, wherein removing, from the data representing the first interrogation signal, the artifact includes applying a filter to the data.
8. The method of clause 7, wherein applying the filter to the data includes convolving the filter and the data in a time domain.
9. The method of clause 7 or 8, wherein applying the filter to the data includes multiplying the filter and the data in a frequency domain.
10. The method of any of clauses 7 to 9, wherein applying the filter to the data includes multiplying the filter and the data in a time domain or convolving the filter and the data in a frequency domain.
11. The method of any of clauses 7 to 10, wherein a stop band of the filter includes the second carrier frequency, wherein a first pass band of the filter includes the first carrier frequency, and wherein the first pass band or a second pass band of the filter includes frequencies below 1 kHz.
12. The method of any of clauses 7 to 11, wherein the filter includes a notch filter configured to reject a portion of the data associated with the second carrier frequency.
13. The method of any of clauses 4 to 12, wherein determining, by analyzing the data representing the first interrogation signal, the transthoracic impedance of the subject includes analyzing an amplitude and a phase of the data representing the first interrogation signal.
14. The method of any of clauses 4 to 13, further including: in response to detecting the artifact, shifting the first interrogation signal from the first carrier frequency to a third carrier frequency, a difference between the second carrier frequency and the third frequency being greater than a difference between the second carrier frequency and the first carrier frequency,
   wherein removing, from the data representing the first interrogation signal, the artifact includes removing the artifact from a portion of the data representing the first interrogation signal having the third carrier frequency.
15. The method of any of clauses 4 to 14, further including: in response to detecting the artifact, causing the first interrogation signal to have the first carrier frequency and a third carrier frequency, wherein removing, from the data representing the first interrogation signal, the artifact includes removing the artifact from a portion of the data representing the first interrogation signal having the first carrier frequency or removing the artifact from a portion of the data representing the first interrogation signal having the third carrier frequency.
16. The method of any of clauses 4 to 15, further including: determining that the subject is moving by detecting a motion artifact in the data representing the first interrogation signal or the transthoracic impedance, the motion artifact being associated with a frequency that is lower than the first carrier frequency and the second carrier frequency.
17. The method of any of clauses 4 to 16, further including: detecting that the subject has received a treatment by detecting a treatment artifact in the data representing the first interrogation signal or the transthoracic impedance, the treatment artifact being associated with a frequency that is lower than the first carrier frequency or the second carrier frequency, the treatment artifact including a chest compression artifact or a ventilation artifact.
18. A medical device, including: a detection circuit configured to detect, from electrodes configured to be disposed on a subject, a first interrogation signal having a first carrier frequency; and a processor configured to: detect, in data representing the first interrogation signal, an artifact associated with a second interrogation signal having a second carrier frequency; remove, from the data representing first interrogation signal, the artifact; and determine, by analyzing the data representing the first interrogation signal, a transthoracic impedance of the subject.
19. The medical device of clause 18, wherein the processor is configured to detect the artifact by: converting the data from a time domain to a frequency domain; in response to converting the data from the time domain to the frequency domain, identifying an amplitude of the data corresponding to the second carrier frequency; and determining that the amplitude of the data is greater than a threshold amplitude.
20. The medical device of clause 18 or 19, wherein the processor is configured to remove the artifact by: generating a filter having a pass band that includes the first carrier frequency and a stop band that includes the second carrier frequency; and applying the filter to the data representing the first interrogation signal.
21. The medical device of any of clauses 18 to 20, further including: a discharge circuit configured to output an electrical shock to the subject, wherein the processor is further configured to determine a voltage or current of the electrical shock by analyzing the transthoracic impedance of the subject.
22. A monitor-defibrillator, including: a detection circuit configured to: output, to electrodes configured to be disposed on a subject, a first interrogation signal having a first carrier frequency and a first current amplitude; detect a voltage of the first interrogation signal; and detect a first electrical shock output to the subject; a treatment circuit configured to output a second electrical shock to the electrodes; a display; and a processor configured to: detect, in data representing the voltage of the first interrogation signal, an artifact associated with a second interrogation signal having a second carrier frequency; determine, by analyzing the second carrier frequency, that the subject is connected to an automated external defibrillator (AED); and in response to determining that the subject is connected to the AED: cause the detection circuit to suppress the first interrogation signal; cause the display to output a notification indicating the AED; and cause the treatment circuit to output the second electrical shock in response to the detection circuit detecting the first electrical shock.
23. The monitor-defibrillator of clause 22, wherein the processor is configured to detect the artifact associated with the second interrogation signal by: converting the data representing the voltage of the first interrogation signal from a time domain to a frequency domain; and in response to converting the data representing the voltage of the first interrogation signal from the time domain to the frequency domain, determining that an amplitude of the data corresponding to the second carrier frequency is above a threshold.
24. The monitor-defibrillator of clause 22 or 23, further including: an input device configured to receive, from a user, an input signal selecting a multi-shock mode,
   wherein the processor is configured to cause the treatment circuit to output the second electrical shock further in response to the input device receiving the input signal.
25. A method, including: receiving data representing a first interrogation signal transmitted through a subject by a first impedance-measuring device, the first interrogation signal having a first carrier frequency; determining that the data representing the first interrogation signal includes an artifact associated with a second interrogation signal having a second carrier frequency; and in response to determining that the data representing the first interrogation signal includes the artifact, determining that the subject is connected to a second impedance-measuring device.
26. The method of clause 25, the artifact is a first artifact, wherein the data representing the first interrogation signal is representative of a first electrical signal detected from electrodes disposed on the subject, wherein the method further includes: in response to determining that the data representing the first interrogation signal includes the artifact: identifying a communication window associated with the second impedance-measuring device, and causing suppression of the first interrogation signal during the communication window; in response to causing suppression of the first interrogation signal, receiving data representing a second electrical signal detected from the electrodes disposed on the subject; and determining that the data representing the second electrical signal includes a second artifact associated with the second interrogation signal having the second carrier frequency, and wherein determining that the subject is connected to the second impedance-measuring device is further in response to determining that the data representing the second electrical signal includes the second artifact.
27. The method of clause 25 or 26, further including: in response to determining that the subject is connected to the second impedance-measuring device, outputting a notification to a user.
28. The method of clause 27, further including: detecting an input signal from the user confirming that the subject is connected to the second impedance-measuring device; and in response to detecting the input signal, suppressing an impedance-dependent function of the first impedance-measuring device.
29. The method of clause 27 or 28, wherein the notification indicates a presence of the second impedance-measuring device or a warning indicating a potential malfunction of the second impedance-measuring device.
30. The method of any of clauses 25 to 29, further including: in response to determining that the subject is connected to the second impedance-measuring device, causing the first impedance-measuring device to suppress the first interrogation signal.
31. The method of any of clauses 25 to 30, further including: in response to determining that the subject is connected to the second impedance-measuring device, causing the first impedance-measuring device to suppress a function utilizing the first interrogation signal, the function including: analyzing an ECG of the subject; removing an artifact from the ECG of the subject; detecting chest compressions performed on the subject; detecting ventilation performed on the subject, detecting spontaneous respiration of the subject, or customizing a parameter of an electrical shock to be administered to the subject.
32. The method of any of clauses 25 to 31, further including: in response to determining that the subject is connected to the second impedance-measuring device, outputting, to the subject, a first electrical shock having a predetermined voltage; determining that the second impedance-measuring device is separated from the subject; in response to determining that the second impedance-measuring device is separated from the subject: determining a transthoracic impedance of the subject by analyzing the first interrogation signal; determining a voltage of a second electrical shock by analyzing the transthoracic impedance; and outputting the second electrical shock.
33. The method of any of clauses 25 to 32, further including: removing, from the data indicative of the first interrogation signal, the artifact; and determining, by analyzing the data indicative of the first interrogation signal, a transthoracic impedance of the subject.
34. The method of any of clauses 25 to 33, further including: determining a type of the second impedance-measuring device by analyzing the artifact; in response to determining the type of the second impedance-measuring device, selecting a subsequent action; and performing the subsequent action.
35. The method of clause 34, wherein selecting the subsequent action includes selecting among: suppressing the first interrogation signal; outputting a notification indicating the type of the second impedance-measuring device; pairing with the second impedance-measuring device; removing the artifact from the first interrogation signal; and deactivating a function utilizing the first interrogation signal.
36. The method of clause 34 or 35, wherein determining the type of the second impedance-measuring device includes: determining a carrier frequency of the second interrogation signal by analyzing the first interrogation signal; and determining the type of the second impedance-measuring device by analyzing the carrier frequency.
37. The method of clause 36, further including: determining, by analyzing the type of the second impedance-measuring device, that there is a risk of damage to the first impedance-measuring device, of damage to the second impedance measuring device, or of damage to the subject.
38. The method of any of clauses 25 to 37, further including: in response to determining that the subject is connected to the second impedance-measuring device, causing the first impedance-measuring device to initiate a self-test.
39. The method of clause 38, wherein causing the first impedance-measuring device to initiate the self-test includes causing the first impedance-measuring device to initiate the self-test in response to receiving a request to power down the first impedance-measuring device.
40. The method of any of clauses 25 to 39, further including: storing, in memory, an indication that the subject is connected to the second impedance-measuring device.
41. A computing device, including: an output device; and a processor configured to: receive data representing a first interrogation signal transmitted through a subject by a first impedance-measuring device, the first interrogation signal having a first carrier frequency; detect, in the data representing the first interrogation signal, an artifact associated with a second interrogation signal having a second carrier frequency; in response to detecting the artifact, determine that the subject is connected to a second impedance-measuring device; and cause the output device to output a notification.
42. The computing device of clause 41, wherein the output device includes a display, a speaker, or a transceiver.
43. The computing device of clause 41 or 42, wherein the processor is configured to detect the artifact in a first portion of the data representing first interrogation signal, the first portion being associated with a first time interval, wherein the processor is further configured to: determine that the artifact is absent from a second portion of the data representing the first interrogation signal, the second portion being associated with a second time interval; and in response to determining that the artifact is absent from the second portion, determine a transthoracic impedance of the subject by analyzing the second portion of the data.
44. The computing device of clause 43, wherein the processor is further configured to:
   determine that the subject is receiving a treatment by detecting a treatment artifact in the second portion of the data representing the first interrogation signal; or determine a voltage or a current of an electrical shock to be delivered to the subject.
45. The computing device of any of clauses 41 to 44, further including: a discharge circuit configured to be electrically coupled to electrodes disposed on the subject, wherein the processor is further configured to: determine that the subject has received a first electrical shock; and in response to determining that the subject has received the first electrical shock, cause the discharge circuit to output a second electrical shock to the electrodes.
46. The computing device of any of clauses 41 to 45, further including: a detection circuit configured to: output the first interrogation signal from electrodes configured to be disposed on the subject; and detect a characteristic of the first interrogation signal from the electrodes, wherein the data representing the first interrogation signal includes data representing an amplitude of the characteristic of the first interrogation signal.
47. The computing device of clause 46, wherein the processor is further configured to: in response to determining that the subject is connected to a second impedance-measuring device: identifying a communication widow associated with the second impedance-measuring device, and causing the detection circuit to suppress the first interrogation signal during the communication window.
48. An external defibrillator, including: a measurement circuit configured to measure a transthoracic impedance of a subject using an impedance interrogation signal applied across electrodes configured to be disposed on skin of the subject; and a processor configured to:
   determine that an impedance-measuring device is electrically connected to the subject and/or in a vicinity of the external defibrillator; in response to determining that the impedance-measuring device is electrically connected to the subject and/or in the vicinity of the external defibrillator: change a present frequency of the impedance interrogation signal to a second frequency that is different than a first frequency used by the impedance-measuring device; or negotiate with the impedance-measuring device such that the impedance-measuring device selects the first frequency and the external defibrillator selects the second frequency; and cause the measurement circuit to measure the transthoracic impedance of the subject using the impedance interrogation signal at the second frequency.
49. The external defibrillator of clause 48, further including a treatment circuit configured to output electrical shocks to the subject, wherein: the external defibrillator is a first external defibrillator configured to operate in a multi-shock mode; the impedance-measuring device is a second external defibrillator; and the processor is further configured to: determine that a manufacturer of the second external defibrillator is different than a manufacturer of the first external defibrillator; in response to determining that the manufacturer of the second external defibrillator is different than the manufacturer of the first external defibrillator, configure the first external defibrillator to output a secondary electrical shock upon expiration of a time interval; and upon expiration of the time interval, cause the treatment circuit to output the secondary electrical shock to the electrodes.
50. The external defibrillator of clause 48 or 49, wherein the processor is configured to change the present frequency of the impedance interrogation signal to the second frequency in response to determining that the impedance-measuring device is electrically connected to the subject and/or in the vicinity of the external defibrillator, and wherein the processor is further configured to: determine that the impedance-measuring device is using the first frequency by analyzing an impedance waveform obtained via the measurement circuit during a period of time when the impedance interrogation signal is not being applied across the electrodes; and determine that the first frequency is within a threshold range of the present frequency of the impedance interrogation signal, wherein changing the present frequency of the impedance interrogation signal to the second frequency is further in response to determining that the first frequency is within the threshold range of the present frequency of the impedance interrogation signal.
51. A method, including: determining, by a medical device, that an impedance-measuring device is electrically connected to a subject and/or in a vicinity of the medical device; in response to determining that the impedance-measuring device is electrically connected to the subject and/or in the vicinity of the medical device, selecting, by the medical device, a second frequency of an impedance interrogation signal that is different than a first frequency used by the impedance-measuring device; and measuring, by the medical device, a transthoracic impedance of the subject by applying the impedance interrogation signal at the second frequency across electrodes disposed on skin of the subject.
52. The method of clause 51, wherein selecting the second frequency of the impedance interrogation signal includes: determining, by the medical device, that the impedance-measuring device is using the first frequency; determining, by the medical device, that the first frequency is within a threshold of a present frequency of the impedance interrogation signal; and in response to determining that the first frequency is within the threshold of the present frequency of the impedance interrogation signal, changing, by the medical device, the present frequency of the impedance interrogation signal to the second frequency.
53. The method of clause 52, wherein determining that the impedance-measuring device is using the first frequency includes analyzing an impedance waveform obtained via a measurement circuit of the medical device during a period of time when the impedance interrogation signal is not being applied across the electrodes.
54. The method of any of clauses 51 to 53, wherein selecting the second frequency of the impedance interrogation signal includes negotiating with the impedance-measuring device such that the impedance-measuring device selects the first frequency and the medical device selects the second frequency.
55. The method of clause 54, wherein negotiating with the impedance-measuring device includes using a wireless communication protocol to negotiate with the impedance-measuring device.
56. The method of clause 54 or 55, wherein negotiating with the impedance-measuring device is further in response to determining that a manufacturer of the impedance-measuring device is the manufacturer of the medical device.
57. The method of any of clauses 51 to 56, wherein the second frequency is selected from among a plurality of predetermined frequencies.
58. The method of any of clauses 51 to 57, wherein determining that the impedance-measuring device is electrically connected to the subject and/or in the vicinity of the medical device includes receiving, by the medical device, a user input signal indicating that the impedance-measuring device is electrically connected to the subject and/or in the vicinity of the medical device.
59. The method of any of clauses 51 to 58, wherein: the medical device includes a defibrillator; and determining that the impedance-measuring device is electrically connected to the subject and/or in the vicinity of the medical device includes receiving, by the defibrillator, a user input signal to activate a multi-shock mode of the defibrillator.
60. A medical device, including: a measurement circuit configured to measure a transthoracic impedance of a subject using an impedance interrogation signal applied across electrodes configured to be disposed on skin of the subject; and a processor configured to:
   determine that an impedance-measuring device is electrically connected to the subject and/or in a vicinity of the medical device; and in response to determining that the impedance-measuring device is electrically connected to the subject and/or in the vicinity of the medical device, select a second frequency of the impedance interrogation signal that is different than a first frequency used by the impedance-measuring device.
61. The medical device of clause 60, wherein, in response to determining that the impedance-measuring device is electrically connected to the subject and/or in the vicinity of the medical device, the processor is further configured to: determine that the impedance-measuring device is using the first frequency; determine that the first frequency is within a threshold range of a present frequency of the impedance interrogation signal; and in response to determining that the first frequency is within the threshold range of the present frequency of the impedance interrogation signal, select the second frequency by changing the present frequency of the impedance interrogation signal to the second frequency.
62. The medical device of clause 61, wherein determining that the impedance-measuring device is using the first frequency includes: analyzing an impedance waveform obtained via the measurement circuit; and classifying the impedance waveform as a waveform associated with multiple impedance interrogation signals at respective frequencies that differ by no more than the threshold range.
63. The medical device of any of clauses 60 to 62, wherein, in response to determining that the impedance-measuring device is electrically connected to the subject and/or in the vicinity of the medical device, the processor is further configured to select the second frequency by negotiating with the impedance-measuring device such that the impedance-measuring device selects the first frequency and the medical device selects the second frequency.
64. The medical device of clause 63, wherein negotiating with the impedance-measuring device is further in response to determining that a manufacturer of the impedance-measuring device is the manufacturer of the medical device.
65. The medical device of any of clauses 60 to 64, wherein the second frequency is selected from among a plurality of predetermined frequencies.
66. The medical device of any of clauses 60 to 65, wherein determining that the impedance-measuring device is electrically connected to the subject and/or in the vicinity of the medical device includes receiving a user input signal indicating that the impedance-measuring device is electrically connected to the subject and/or in the vicinity of the medical device.
67. The medical device of any of clauses 60 to 66, wherein the medical device includes a first external defibrillator and the impedance-measuring device includes a second external defibrillator.
68. An external defibrillator, including: a treatment circuit configured to output an electrical shock to a subject; a measurement circuit configured to measure a transthoracic impedance of the subject using an impedance interrogation signal applied across electrodes configured to be disposed on skin of the subject; and a processor configured to: determine that an impedance-measuring device is electrically connected to the subject and/or in a vicinity of the external defibrillator; in response to determining that the impedance-measuring device is electrically connected to the subject and/or in the vicinity of the external defibrillator, disable one or more functions of the external defibrillator that utilize the transthoracic impedance of the subject; and cause the treatment circuit to output an electrical shock to the electrodes while the one or more functions are disabled.
69. The external defibrillator of clause 68, wherein the one or more functions include: a first function of outputting recommendations to administer one or more electrical shocks to the subject or to not administer any shocks to the subject; a second function of detecting motion of the subject by detecting an artifact in the impedance interrogation signal; a third function of determining a voltage of the electrical shock by analyzing the impedance interrogation signal; or a fourth function of determining a duration of the electrical shock by analyzing the impedance interrogation signal.
70. The external defibrillator of clause 68 or 69, further including a display, wherein the processor is further configured to cause the display to output a visual alert indicating that the one or more functions have been disabled.
71. A method, including: determining, by a medical device configured to measure an impedance of a subject, that an impedance-measuring device is electrically connected to the subject and/or in a vicinity of the medical device; and in response to determining that the impedance-measuring device is electrically connected to the subject and/or in the vicinity of the medical device, disabling, by the medical device, one or more functions of the medical device that utilize the impedance of the subject.
72. The method of clause 71, further including: determining, by the medical device, that the impedance-measuring device is using a first frequency; and determining, by the medical device, that the first frequency is within a threshold range of a present frequency of an impedance interrogation signal that the medical device is configured to apply across electrodes disposed on skin of the subject to measure the impedance, wherein disabling the one or more functions of the medical device is further in response to determining that the first frequency is within the threshold range of the present frequency of the impedance interrogation signal.
73. The method of clause 72, wherein determining that the impedance-measuring device is using the first frequency includes analyzing an impedance waveform obtained via a measurement circuit of the medical device during a period of time when the impedance interrogation signal is not being applied across the electrodes.
74. The method of clause 72 or 73, wherein determining that the impedance-measuring device is using the first frequency includes: analyzing an impedance waveform obtained via a measurement circuit of the medical device; and classifying the impedance waveform as a waveform associated with multiple impedance interrogation signals at respective frequencies that differ by no more than the threshold.
75. The method of any of clauses 71 to 74, wherein the impedance is a transthoracic impedance.
76. The method of any of clauses 71 to 75, further including: indicating, in a record associated with the subject, that the impedance-measuring device was determined to be electrically connected to the subject and/or in the vicinity of the medical device.
77. The method of any of clauses 71 to 76, further including causing, by the medical device, an output device of the medical device to output an alert indicating that the one or more functions have been disabled.
78. The method of any of clauses 71 to 77, further including preventing, by the medical device, a measurement circuit of the medical device from using an impedance interrogation signal to measure the impedance of the subject while the impedance-measuring device is electrically connected to the subject and/or in the vicinity of the medical device.
79. The method of any of clauses 71 to 78, further including, in response to determining that the impedance-measuring device is electrically connected to the subject and/or in the vicinity of the medical device: causing, by the medical device, an output device of the medical device to output: an alert indicating that another impedance-measuring device electrically connected to the subject and/or in the vicinity of the medical device may cause interference with measurements of the impedance of the subject; and a prompt for a user of the medical device to disable the one or more functions; and receiving, via an input device of the medical device, and in association with the prompt, a user input signal to disable the one or more functions, wherein disabling the one or more functions of the medical device is further in response to receiving the user input signal.
80. The method of any of clauses 71 to 79, wherein: the medical device includes a defibrillator; and the one or more functions include: a first function of outputting recommendations to administer one or more electrical shocks to the subject or to not administer any electrical shocks to the subject; a second function of detecting motion of the subject; or a third function of implementing voltage compensation of defibrillation waveforms.
81. The method of any of clauses 71 to 80, wherein: the medical device includes a defibrillator; and determining that the impedance-measuring device is electrically connected to the subject and/or in the vicinity of the medical device includes receiving, by the defibrillator, a user input signal to activate a multi-shock mode of the defibrillator.
82. A medical device, including: a measurement circuit configured to measure a transthoracic impedance of a subject using an impedance interrogation signal applied across electrodes configured to be disposed on skin of the subject; and a processor configured to:
   determine that an impedance-measuring device is electrically connected to the subject and/or in a vicinity of the medical device; and in response to determining that the impedance-measuring device is electrically connected to the subject and/or in the vicinity of the medical device, disable one or more functions of the medical device that utilize the transthoracic impedance of the subject.
83. The medical device of clause 82, wherein the processor is further configured to:
   determine that the impedance-measuring device is using a first frequency; and determine that the first frequency is within a threshold range of a present frequency of the impedance interrogation signal, wherein disabling the one or more functions of the medical device is further in response to determining that the first frequency is within the threshold range of the present frequency of the impedance interrogation signal.
84. The medical device of clause 83, wherein determining that the impedance-measuring device is using the first frequency includes: analyzing an impedance waveform obtained via the measurement circuit; and classifying the impedance waveform as a waveform associated with multiple impedance interrogation signals at respective frequencies that differ by no more than the threshold range.
85. The medical device of any of clauses 82 to 84, further including an output device, wherein the processor is further configured to cause the output device to output an alert indicating that the one or more functions have been disabled.
86. The medical device of any of clauses 82 to 85, wherein the processor is further configured to prevent the measurement circuit from using the impedance interrogation signal to measure the transthoracic impedance of the subject while the impedance-measuring device is electrically connected to the subject and/or in the vicinity of the medical device.
87. The medical device of any of clauses 82 to 86, further including an input device and an output device, wherein the processor is further configured to, in response to determining that the impedance-measuring device is electrically connected to the subject and/or in the vicinity of the medical device: cause the output device to output: an alert indicating that another impedance-measuring device electrically connected to the subject and/or in the vicinity of the medical device may cause interference with measurements of the transthoracic impedance of the subject; and a prompt for a user of the medical device to disable the one or more functions; and receive, via the input device, and in association with the prompt, a user input signal to disable the one or more functions, wherein disabling the one or more functions of the medical device is further in response to receiving the user input signal.
88. The medical device of any of clauses 82 to 87, wherein determining that the impedance-measuring device is electrically connected to the subject and/or in the vicinity of the medical device includes receiving a user input signal indicating that the impedance-measuring device is electrically connected to the subject and/or in the vicinity of the medical device.
89. The medical device of any of clauses 82 to 88, wherein the medical device includes a first external defibrillator and the impedance-measuring device includes a second external defibrillator.

### CONCLUSION

The features disclosed in the foregoing description, or the following claims, or the accompanying drawings, expressed in their specific forms or in terms of a means for performing the disclosed function, or a method or process for attaining the disclosed result, as appropriate, may, separately, or in any combination of such features, be used for realizing implementations of the disclosure in diverse forms thereof.

As will be understood by one of ordinary skill in the art, each implementation disclosed herein can comprise, consist essentially of or consist of its particular stated element, step, or component. Thus, the terms "include" or "including" should be interpreted to recite: "comprise, consist of, or consist essentially of." The transition term "comprise" or "comprises" means has, but is not limited to, and allows for the inclusion of unspecified elements, steps, ingredients, or components, even in major amounts. The transitional phrase "consisting of" excludes any element, step, ingredient or component not specified. The transition phrase "consisting essentially of" limits the scope of the implementation to the specified elements, steps, ingredients or components and to those that do not materially affect the implementation. As used herein, the term "based on" is equivalent to "based at least partly on," unless otherwise specified.

Unless otherwise indicated, all numbers expressing quantities, properties, conditions, and so forth used in the specification and claims are to be understood as being modified in all instances by the term "about." Accordingly, unless indicated to the contrary, the numerical parameters set forth in the specification and attached claims are approximations that may vary depending upon the desired properties sought to be obtained by the present disclosure. At the very least, and not as an attempt to limit the application of the doctrine of equivalents to the scope of the claims, each numerical parameter should at least be construed in light of the number of reported significant digits and by applying ordinary rounding techniques. When further clarity is required, the term "about" has the meaning reasonably ascribed to it by a person skilled in the art when used in conjunction with a stated numerical value or range, i.e. denoting somewhat more or somewhat less than the stated value or range, to within a range of ±20% of the stated value; ±19% of the stated value; ±18% of the stated value; ±17% of the stated value; ±16% of the stated value; ±15% of the stated value; ±14% of the stated value; ±13% of the stated value; ±12% of the stated value; ±11% of the stated value; ±10% of the stated value; ±9% of the stated value; ±8% of the stated value; ±7% of the stated value; ±6% of the stated value; ±5% of the stated value; ±4% of the stated value; ±3% of the stated value; ±2% of the stated value; or ±1% of the stated value.

Notwithstanding that the numerical ranges and parameters setting forth the broad scope of the disclosure are approximations, the numerical values set forth in the specific examples are reported as precisely as possible. Any numerical value, however, inherently contains certain errors necessarily resulting from the standard deviation found in their respective testing measurements.

The terms "a," "an," "the" and similar referents used in the context of describing implementations (especially in the context of the following claims) are to be construed to cover both the singular and the plural, unless otherwise indicated herein or clearly contradicted by context. Recitation of ranges of values herein is merely intended to serve as a shorthand method of referring individually to each separate value falling within the range. Unless otherwise indicated herein, each individual value is incorporated into the specification as if it were individually recited herein. All methods described herein can be performed in any suitable order unless otherwise indicated herein or otherwise clearly contradicted by context. The use of any and all examples, or exemplary language (e.g., "such as") provided herein is intended merely to better illuminate implementations of the disclosure and does not pose a limitation on the scope of the disclosure. No language in the specification should be construed as indicating any nonclaimed element essential to the practice of implementations of the disclosure.

Groupings of alternative elements or implementations disclosed herein are not to be construed as limitations. Each group member may be referred to and claimed individually or in any combination with other members of the group or other elements found herein. It is anticipated that one or more members of a group may be included in, or deleted from, a group for reasons of convenience and/or patentability. When any such inclusion or deletion occurs, the specification is deemed to contain the group as modified thus fulfilling the written description of all Markush groups used in the appended claims.

Certain implementations are described herein, including the best mode known to the inventors for carrying out implementations of the disclosure. Of course, variations on these described implementations will become apparent to those of ordinary skill in the art upon reading the foregoing description. The inventors expect skilled artisans to employ such variations as appropriate, and the inventors intend for implementations to be practiced otherwise than specifically described herein. Accordingly, the scope of this disclosure includes all modifications and equivalents of the subject matter recited in the claims appended hereto as permitted by applicable law. Moreover, any combination of the above-described elements in all possible variations thereof is encompassed by implementations of the disclosure unless otherwise indicated herein or otherwise clearly contradicted by context.

## Claims

1. A method, comprising:
receiving data representing a first interrogation signal transmitted through a subject by a first impedance-measuring device, the first interrogation signal having a first carrier frequency;
determining that the data representing the first interrogation signal comprises an artifact associated with a second interrogation signal having a second carrier frequency; and
in response to determining that the data representing the first interrogation signal comprises the artifact, determining that the subject is connected to a second impedance-measuring device.

2. The method of claim 1, the artifact is a first artifact, wherein the data representing the first interrogation signal is representative of a first electrical signal detected from electrodes disposed on the subject,
wherein the method further comprises:
in response to determining that the data representing the first interrogation signal comprises the artifact, causing suppression of the first interrogation signal;
in response to causing suppression of the first interrogation signal, receiving data representing a second electrical signal detected from the electrodes disposed on the subject; and
determining that the data representing the second electrical signal comprises a second artifact associated with the second interrogation signal having the second carrier frequency, and
wherein determining that the subject is connected to the second impedance-measuring device is further in response to determining that the data representing the second electrical signal comprises the second artifact.

3. The method of claim 1 or 2, further comprising:
in response to determining that the subject is connected to the second impedance-measuring device, outputting a notification to a user.

4. The method of claim 3, further comprising:
detecting an input signal from the user confirming that the subject is connected to the second impedance-measuring device; and
in response to detecting the input signal, suppressing an impedance-dependent function of the first impedance-measuring device.

5. The method of claim 3 or 4, wherein the notification indicates a presence of the second impedance-measuring device or a warning indicating a potential malfunction of the second impedance-measuring device.

6. The method of any of claims 1 to 5, further comprising:
in response to determining that the subject is connected to the second impedance-measuring device:
identifying a communication window associated with the second impedance-measuring device; and
causing the first impedance-measuring device to suppress the first interrogation signal during the communication window.

7. The method of any of claims 1 to 6, further comprising:
in response to determining that the subject is connected to the second impedance-measuring device, causing the first impedance-measuring device to suppress a function utilizing the first interrogation signal, the function comprising:
analyzing an ECG of the subject;
removing an artifact from the ECG of the subject;
detecting chest compressions performed on the subject;
detecting ventilation performed on the subject,
detecting spontaneous respiration of the subject, or
customizing a parameter of an electrical shock to be administered to the subject.

8. The method of any of claims 1 to 7, further comprising:
removing, from the data indicative of the first interrogation signal, the artifact; and
determining, by analyzing the data indicative of the first interrogation signal, a transthoracic impedance of the subject.

9. The method of any of claims 1 to 8, further comprising:
determining a type of the second impedance-measuring device by analyzing the artifact;
in response to determining the type of the second impedance-measuring device, selecting a subsequent action; and
performing the subsequent action.

10. The method of claim 9, wherein selecting the subsequent action comprises selecting among:
suppressing the first interrogation signal;
outputting a notification indicating the type of the second impedance-measuring device;
pairing with the second impedance-measuring device;
removing the artifact from the first interrogation signal; and
deactivating a function utilizing the first interrogation signal.

11. The method of claim 9 or 10, wherein determining the type of the second impedance-measuring device comprises:
determining a carrier frequency of the second interrogation signal by analyzing the first interrogation signal; and
determining the type of the second impedance-measuring device by analyzing the carrier frequency, and
wherein the method further comprises:
determining, by analyzing the type of the second impedance-measuring device, that there is a risk of damage to the first impedance-measuring device, of damage to the secondimpedance measuring device, or of damage to the subject.

12. The method of any of claims 1 to 11, further comprising:
in response to determining that the subject is connected to the second impedance-measuring device, causing the first impedance-measuring device to initiate a self-test.

13. The method of claim 12, wherein causing the first impedance-measuring device to initiate the self-test comprises causing the first impedance-measuring device to initiate the self-test in response to receiving a request to power down the first impedance-measuring device.

14. The method of any of claims 1 to 13, further comprising:
storing, in memory, an indication that the subject is connected to the second impedance-measuring device.

15. A computing device, comprising:
an output device; and
a processor configured to:
receive data representing a first interrogation signal transmitted through a subject by a first impedance-measuring device, the first interrogation signal having a first carrier frequency;
detect, in the data representing the first interrogation signal, an artifact associated with a second interrogation signal having a second carrier frequency;
in response to detecting the artifact, determine that the subject is connected to a second impedance-measuring device; and
cause the output device to output a notification.
